# EUROPÄISCHE PATENTSCHRIFT

(11) **EP 1 583 967 B1**
(45) Veröffentlichungstag und Bekanntmachung des Hinweises auf die Patenterteilung: **27.06.2007**
(21) Anmeldenummer: 03795968.1
(22) Anmeldetag: 24.12.2003
(51) Int. Cl.: G01N 33/569, A61P 31/22

(54) **VERFAHREN ZUR INHIBIERUNG DER REPLIKATION VON HERPESVIREN**
METHOD FOR INHIBITING THE REPLICATION OF HERPES VIRUSES
PROCEDE POUR INHIBER LA REPLICATION DE VIRUS HERPETIQUES

(30) Priorität: 07.01.2003 DE 10300109
(43) Veröffentlichungstag der Anmeldung: 12.10.2005
(73) Patentinhaber: AiCuris GmbH & Co. KG, 42117 Wuppertal (DE)
(72) Erfinder: BETZ, Ulrich, 42119 Wuppertal (DE); HEWLETT, Guy, 42327 Wuppertal (DE); KLEYMANN, Gerald, 32107 Bad-Salzuflen (DE); LAMPE, Thomas, 40223 Düsseldorf (DE); LIN, Tse-I, 2800 Mechelen (BE); NIKOLIC, Susanne, 40789 Monheim (DE); REEFSCHLÄGER, Jürgen, 26125 Oldenburg (DE); WUNBERG, Tobias, 42699 Solingen (DE); ZIMMERMANN, Holger, 42113 Wuppertal (DE); ZUMPE, Franz, 47918 Tönisvort (DE); BENDER, Wolfgang, 51399 Burscheid (DE); HENNINGER, Kerstin, 42115 Wuppertal (DE); JENSEN, Axel, 42553 Velbert (DE); KELDENICH, Jörg, 42113 Wuppertal (DE); SCHOHE-LOOP, Rudolf, 42327 Wuppertal (DE)
(74) Vertreter: Weller, Wolfgang
(86) Internationale Anmeldenummer: PCT/EP2003/014883
(87) Internationale Veröffentlichungsnummer: WO 2004/060860

(56) Entgegenhaltungen:
- EP-A- 0 860 700
- WO-A-00/09158
- WO-A-03/097595
- US-A- 6 054 472
- US-A1- 2001 007 877
- THOMSEN DARRELL R ET AL: "Assembly of herpes simplex virus (HSV) intermediate capsids in insect cells infected with recombinant baculoviruses expressing HSV capsid proteins" JOURNAL OF VIROLOGY, Bd. 68, Nr. 4, 1994, Seiten 2442-2457, XP002287245 ISSN: 0022-538X
- DESAI PRASHANT ET AL: "Mutations in herpes simplex virus type 1 genes encoding VP5 and VP23 abrogate capsid formation and cleavage of replicated DNA" JOURNAL OF VIROLOGY, Bd. 67, Nr. 3, 1993, Seiten 1357-1364, XP002287246 ISSN: 0022-538X
- PLOTKIN S A ET AL: "CANDIDATE CYTOMEGALOVIRUS STRAIN FOR HUMAN VACCINATION" INFECTION AND IMMUNITY, Bd. 12, Nr. 3, 1975, Seiten 521-527, XP002287247 ISSN: 0019-9567
- WELLER S K ET AL: "GENETIC AND PHENOTYPIC CHARACTERIZATION OF MUTANTS IN FOUR ESSENTIAL GENES THAT MAP TO THE LEFT HALF OF HSV-1 U-L DNA" VIROLOGY, Bd. 161, Nr. 1, 1987, Seiten 198-210, XP001182178 ISSN: 0042-6822
- SHERMAN G ET AL: "CHARACTERIZATION OF INTRANUCLEAR CAPSIDS MADE BY TS MORPHOGENIC MUTANTS OF HSV-1" VIROLOGY, Bd. 163, Nr. 2, 1988, Seiten 471-480, XP001182182 ISSN: 0042-6822

## Beschreibung

Die Erfindung betrifft ein Verfahren um Identifizieren von Verbindungen welche die Replikation von Herpesviren inhibieren.

Die Familie der Herpesviren gliedert sich in die drei Sub-Familien Alpha-Herpesviren (z.B. Herpes simplex Virus Typ 1 und 2; HSV1 und HSV2), Beta-Herpesviren (z.B. Cytomegalievirus; HCMV) and Gamma-Herpesviren (z.B. Epstein-Barr Virus; EBV).

Infektionen mit Herpes-Viren manifestieren sich je nach Virustyp in Erkrankungen unterschiedlicher Organe wie der Haut, des lymphatischen Systems oder des zentralen Nervensystems.

Infektionen mit dem Beta-Herpesvirus HCMV erfolgen meist während der Kindheit and verlaufen in der Regel sub-klinisch. Die Durchseuchungsrate bei Erwachsenen ist daher weltweit sehr hoch (je nach untersuchter Population bis zu 90%).

Innerhalb der Familie der Herpesviren führt das Cytomegalievirus zur höchsten Sterblichkeitsrate unter immungeschwächten Patienten. Dies ist darauf zurückzuführen, dass Cytomegalieviren bei diesen Personen lebensbedrohende, generalisierte Erkrankungen, besonders Pneumonien verursachen.

Bei schwangeren Frauen können Infektionen mit HCMV schwere Schädigungen des Kindes zur Folge haben.

Die Viruspartikel der Herpesviren haben Durchmesser von ca. 150 bis 200 mm and setzen sich aus verschiedenen für das Virus essentiellen Strukturproteinen zusammen. Im Inneren der Partikel findet man das Virus-Core - eine fibrilläre Proteinmatrix, mit der das doppelsträngige, lineare DNA-Genom assoziiert ist. Das Core ist von einem ikosaedrischen Capsid umgeben, das aus 162 Capsomeren besteht. Das Hauptcapsidprotein (Major-Capsid-Protein, MCP) des humanen Cytomegalievirus wird als UL86 bezeichnet.

Das virale Protein UL80 wird zu mindestens drei verschiedenen Proteinen prozessiert, die bei der Kapsidreifung eine Rolle spielen. Die häufigste Form ist dabei das Assembly-Protein AP.

Die Bildung von Viruspartikeln - zunächst werden B-Kapside gebildet - wird kontrolliert von einem Vorläufer-Komplex aus UL86 und AP, der für die Translokation des MCP (UL86) in den Zellkern verantwortlich ist. Im Zellkern unterstützt AP die Bildung von Strukturen, die ein internes Gerüst innerhalb der B-Capside bilden. In die fertigen B-Kapside wird die virale DNA verpackt, wobei AP aus den Viruspartikeln ausgeschleust wird. Die DNA-haltigen infektiösen Viruspartikel werden auch als C-Kapside bezeichnet.

Zur Prophylaxe einer HCMV-Infektion steht zur Zeit kein Impfstoff zur Verfügung. Für die Therapie der HCMV-Infektion wird hauptsächlich Ganciclovir eingesetzt, welches jedoch starke Nebenwirkungen verursacht.

Daraus ergibt sich ein lange bestehendes Bedürfnis nach einer verbesserten and vor allem verträglicheren HCMV-Therapie. Die Nachfrage nach einer verbesserten HCMV-Therapie wird zudem dadurch verstärkt, dass zum einen in zunehmendem Maße Organtransplantationen durchgeführt werden und außerdem die Zahl der HIV-Infizierten stetig steigt. Bei beiden Patientengruppen kann es aufgrund der Immunsuppression zu Komplikationen aufgrund einer HCMV-Reaktivierung oder HCMV-Infektion kommen. Daher ist hier eine verbesserte HCMV-Therapie dringend notwendig.

Ziel ist es, die Replikation von Herpesviren zu inhibieren. Dies ist möglich durch Verbindungen, welche auf das Major Capsid Protein zielen and dabei die Bildung von C-Kapsiden, nicht aber von B-Kapsiden inhibieren. Viren, selektiert auf Resistenz gegen diese Verbindung zeigen eine oder mehrere Mutationen im für das Major Capsid Protein kodierenden Gen.

In der EP 0 860 700 A2 wird ein Verfahren zum Identifizieren von antiviralen Verbindungen beschrieben, die mit dem Major Capsid Protein VP5 aus HSV-1 interagieren.

In der US 2001/0007877 A1 wird ein Verfahren beschrieben, in dem die Inhibition der Komplexbildung zwischen dem Major Capsid Protein aus Cytomegalievirus und dem Scaffolding-Protein-Peptid durch eine Testsubstanz untersucht wird.

Eine Aufgabe der vorliegenden Anmeldung ist es, ein Verfahren zur Identifizierung von Verbindungen mit diesem neuen Wirkmechanismus mit Aktivität gegen Herpesviren bereitzustellen.

Diese Aufgabe wird gelöst durch ein Verfahren, gekennzeichnet dadurch, dass
i) Herpesviren mit Testverbindungen in Kontakt gebracht werden,
ii) resistente Herpesviren ausgelesen werden,
iii) das für das Major Capsid Protein kodierende Gen dieser resistenten Herpesviren sequenziert und die resultierende Proteinsequenz des Major Capsid Proteins abgeleitet wird, und
iv) solche Verbindungen ausgewählt werden, bei denen resistente Herpesviren mit einer oder mehreren Aminosäuresubstitutionen im Major Capsid Protein auftreten.

Unter einem Verfahren zum Auswählen von Verbindungen mit Aktivität gegen Herpesviren wird im Rahmen der vorliegenden Erfindung ein Verfahren verstanden, bei dem neue oder an sich bekannte Verbindungen auf ihre Aktivität gegen Herpesviren untersucht werden.

Herpesviren sind hierbei beispielsweise Beta-Herpesviren, insbesondere das humane Cytomegalievirus, insbesondere die HCMV-Stämme Ad169 (ATCC VR-538) oder Davis (ATCC VR-807). Nicht bevorzugt ist die Verwendung des Stamms HCMV-Towne (ATCC VR-977), da dieser bereits eine Mutation im UL86 Gen trägt (P1189T), welche zu einer entsprechenden Resistenz gegen die nach dem hier beschriebenen Wirkmechanismus wirkenden Substanzen führt.

Überraschenderweise wurden Testverbindungen gefunden, welche sich durch einen einzigartigen Wirkmechanismus auszeichnen. Bei Kultivierung von HCMV unter Substanzdruck wird die Bildung von B-Capsiden zugelassen, wohingegen die Formierung von infektiösen C-Capsiden verhindert wird. Die Aufrechterhaltung der Bildung von V-Kapsiden unter der antiviralen Behandlung könnte insofern einen Vorteil darstellen, als dass B-Capside als Immunogen im Verlauf des viralen Replikationszyklus zunächst präsent bleiben, was sich zum Beispiel im Falle eines wiedererstarkten Immunsystems vorteilhaft für eine daraus resultierende spezifische Immunabwehrreaktion auswirken könnte.

Bei der Sequenzanalyse von Cytomegalieviren, die resistent gegenüber den Testverbindungen sind, wurden überraschenderweise nur Mutationen im Capsidprotein UL86 gefunden.

Da diese mutierten Viren unter Substanzdruck wachsen können, lässt sich daraus schließen, dass die Testsubstanz bei den sensitiven Wildtyp-Viren gerade an diesem Protein angreift. Elektronenmikroskopische und molekularbiologische Untersuchungen zeigen, dass durch die Substanzen die Encapsidierung viraler DNA and damit die Bildung von C-Capsiden inhibiert wird. Nicht beeinträchtigt ist die Replikation der viralen DNA und die Bildung von DNA-freien unreifen Capsiden (B-Capsiden). Im Vergleich zu dem als HCMV-Medikament in der Klinik eingesetzten DNA-Replikationsinhibitor Ganciclovir, erfolgt unter Einfluss der erfindungsgemäßen Substanzen keine Inhibition der DNA Synthese und Expression der späten HCMV Gene. Die in UL86 gefundenen Mutationen treten bevorzugt in zwei Bereichen auf. Der erste Bereich erstreckt sich von der Aminosäureposition 435 bis 689 und der zweite Bereich von Aminosäureposition 1189 bis 1338 (siehe Tabelle 2).

Die antivirale Wirkung kann zum einen durch direkte Interaktion der Substanz mit UL86 zustande kommen oder aber auch über einen indirekten Effekt auf UL86 wirken.

Nach diesem neuen und überraschenden Wirkmechanismus wirkende antivirale Substanzen können noch durch weitere Methoden erhalten werden, wie z.B. Molecular Modelling mit Hilfe der dreidimensionalen Struktur eines Major Capsid Proteins, Molecular Modelling ausgehend von bekannten UL86-Inhibitoren usw. Dabei handelt es sich um dem Fachmann wohlbekannte Methoden.

Über die Funktionen und Interaktionen von UL86 während der Kapsidreifung und DNA Verpackung ist wenig bekannt. (Wood et al., J.Virol, 1997, 71, 179-190).

Die zur Zeit erhältlichen Medikamente gegen HCMV sind aufgrund starker Nebenwirkungen nicht ideal. Hochdurchsatztestungen von großen Substanzbanken haben in der letzten Zeit zu Inhibitoren geführt, die an weiteren viralen Targets angreifen (Wathen, Rev Med Virol, 2002, 12, 167-178). Der hier beschriebene Wirkmechanismus zeigt eine überraschende neue Option auf, wie die Replikation von Herpesviren mit Hilfe von Verbindungen inhibiert werden kann.

Die Identifizierung von Major Capsid Protein bindenden Verbindungen kann geschehen durch Aufreinigung von Capsiden, rekombinante Expression von Major Capsid Protein oder Teilfragmenten des Major Capsid Proteins und Messung von an das Protein bzw. Proteinfragment bindenden Substanzen (z.B. HPLC, Verdrängung fluoreszierender Peptide, Verdrängung von Aptameren, diverse spektroskopische Methoden etc.), welche dem Fachmann wohlbekannt sind.

Weiterhin ist die Identifizierung möglich durch folgendes Verfahren mit Testsubstanzen: Unter Testverbindungen werden solche Verbindungen verstanden, die auf ihre Aktivität gegen Herpesviren untersucht werden sollen. Dies können neue oder an sich bekannte Verbindungen sein. Diese werden mit den Herpesviren in Kontakt gebracht. Dies geschieht bevorzugterweise durch Kultivierung von HCMV in 384-well Gewebekulturplatten. Dazu werden suszeptible Zellen, bevorzugterweise humane Fibroblasten, in Gewebekulturgefäßen ausgesät. Bevorzugterweise werden 5 x 10³ Zellen pro well auf einer 96-well-Platte eingesetzt und mit HCMV infiziert (bevorzugterweise mit einer moi von 0,03).

Die Infektionen werden unter verschiedenen Substanzkonzentrationen (bevorzugterweise bei Konzentrationen von 0,005 bis 250 µM) kultiviert, bis in der Viruskontrolle ein deutlicher CPE zu erkennen ist (in der Regel nach 6 Tagen). Dann kann aus den anderen Substanzkonzentrationen der IC₅₀ bestimmt werden. Wirksame Substanzen zeichnen sich durch einen IC₅₀-Wert aus, der bevorzugterweise < 1µM ist und zudem einen SI > 10 aufweist.

Viren, die gegen wirksame Substanzen resistent sind, können wie folgt angezüchtet werden:

Bevorzugterweise erfolgt die Kultivierung von HCMV wiederum in 96-well Gewebekulturplatten. Dazu werden suszeptible Zellen, bevorzugterweise humane Fibroblasten, in Gewebekulturgefäßen ausgesät. Bevorzugterweise werden 5 x 10³ Zellen pro well auf einer 96-well-Platte eingesetzt und mit HCMV infiziert (bevorzugterweise mit einer moi von 0,03). Die Infektionen werden jetzt unter Substanzdruck kultiviert, der dem 10-fachen IC₅₀ Wert der Substanz entspricht.

Zellkulturen, die einen cytopathischen Effekt (CPE) vergleichbar einer Virusinfektion ohne Substanzdruck aufweisen, werden weiter analysiert, d.h. die Viren werden auf frischen Zellkulturen passagiert und weiter unter Substanzdruck kultiviert. Viren, die unter Sübstanzdruck weiter wachsen und einen RI (Resistenzindex) > 5 aufweisen, werden als resistente Viren bezeichnet.

Die DNA der resistenten Viren wird isoliert und anschließend wird die Nukleotidsequenz des für das MCP kodierenden Gens (UL86) bestimmt und mit der Sequenz des Ausgangsvirus (Wildtyp-Virus der gegenüber der Substanz sensitiv ist) verglichen. Resistente Viren, die Mutationen in der für das Major Capsid Protein (UL86) resultierenden Aminosäureseqeunz aufweisen, identifizieren eine Substanz, die als UL86-Inhibitor eingesetzt werden kann.

In den meisten Screens zur Identifizierung von anti-HCMV Wirkstoffen wird der seit langem bekannte und sehr etablierte Laborstamm HCMV Towne verwendet. Nach dem erfindungsgemäßen Wirkmechanismus wirkende Substanzen sind hiermit nicht oder nur sehr schwer aufzufinden. Daher ist eine wichtige Methode zur Therapie und Prophylaxe von HCMV-Infektionen bisher völlig unberücksichtigt geblieben, welche in diesem Patent beschrieben wird. In Screens zur Identifizierung von anti-HCMV Wirkstoffen können Substanzen ausgewählt werden, welche die Replikation von HCMVTowne nicht oder nur unzureichen hemmen, aber die Replikation von HCMV Wildtyp Stämmen unterdrücken. Diese Wirkstoffe können dann zur Therapie und Prophylaxe von HCMV-Infektionen eingesetzt werden.

Bevorzugt sind
[A] Verbindungen der allgemeinen Formel (Ia), 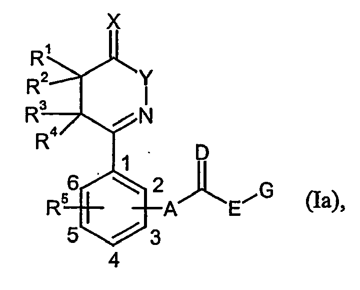 in welcher
   A über die Positionen 2, 3, 5 oder 6 an den Aromaten gebunden ist und
   - A: für Sauerstoff oder NR⁶ steht,
   - E: für Sauerstoff, CR⁹R¹⁰ oder NR⁷ steht,
   - Y: für Sauerstoff oder NR⁸ steht,
   - D und X: gleich oder verschieden sind und jeweils für Sauerstoff oder Schwefel stehen,
   - G: für Wasserstoff steht,
   oder
   - G: für C₆-C₁₀-Aryl steht, wobei C₆-C₁₀-Aryl gegebenenfalls substituiert sein kann mit bis zu drei Substituenten ausgewählt aus der Gruppe bestehend aus Halogen, Hydroxy, Nitro, Cyano, C₁-C₆-Alkoxy, Hydroxycarbonyl, C₁-C₆-Alkoxycarbonyl, Amino, mono- oder di-C₁-C₆-Alkylamino, mono- oder di-C₁-C₆-Alkylaminocarbonyl und C₁-C₆-Alkyl,
   worin
   C₁-C₆-Alkoxy, C₁-C₆-Alkoxycarbonyl, mono- oder di-C₁-C₆-Alkylamino, mono- oder di-C₁-C₆-Alkylaminocarbonyl oder C₁-C₆-Alkyl gegebenenfalls substituiert sein können mit bis zu drei Substituenten ausgewählt aus der Gruppe bestehend aus Halogen, Hydroxy, C₁-C₆-Alkoxy, Amino, mono- oder di-C₁-C₆-Alkylamino, Hydroxycarbonyl, C₁-C₆-Alkoxycarbonyl, mono- oder di-C₁-C₆-Alkylaminocarbonyl und C₆-C₁₀-Aryl,
   oder
   - G: für C₆-C₁₀-Aryl steht, wobei C₆-C₁₀-Aryl gegebenenfalls substituiert sein kann mit Phenyl,
   worin
   Phenyl gegebenenfalls substituiert sein kann mit bis zu drei Substituenten ausgewählt aus der Gruppe bestehend aus Halogen, Hydroxy, C₁-C₆-Alkoxy, Amino, mono- oder di-C₁-C₆-Alkylamino, Hydroxycarbonyl, C₁-C₆-Alkoxycarbonyl, mono- oder di-C₁-C₆-Alkylaminocarbonyl und C₁-C₆-Alkyl,
   worin
   C₁-C₆-Alkyl seinerseits gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, Hydroxycarbonyl, C₁-C₆-Alkoxycarbonyl und mono- oder di-C₁-C₆-Alkylaminocarbonyl,
   oder
   - G: für C₆-C₁₀-Aryl steht, wobei C₆-C₁₀-Aryl gegebenenfalls substituiert sein kann mit Phenyl,
   worin
   Phenyl gegebenenfalls substituiert sein kann mit C₅-C₆-Heteroaryl oder C₅-C₇-Heterocyclyl,
   worin
   C₅-C₆-Heteroaryl oder C₅-C₇-Heterocyclyl ihrerseits gegebenenfalls substituiert sein können mit bis zu drei Substituenten ausgewählt aus der Gruppe bestehend aus Halogen, C₁-C₆-Alkyl, C₁-C₆-Alkoxy, Amino, mono- oder di-C₁-C₆-Alkylamino, Hydroxycarbonyl, C₁-C₆-Alkoxycarbonyl und mono- oder di-C₁-C₆-Alkylaminocarbonyl,
   oder
   - G: für C₆-C₁₀-Aryl steht, wobei C₆-C₁₀-Aryl gegebenenfalls substituiert sein kann mit einer Gruppe der folgenden Formel 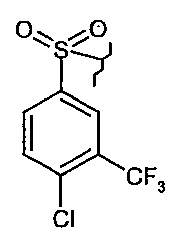
   oder
   - G: für C₅-C₁₀-Heteroaryl oder C₅-C₇-Heterocyclyl steht, wobei C₅-C₁₀-Heteroaryl oder C₅-C₇-Heterocyclyl gegebenenfalls substituiert sein kann mit bis zu drei Substituenten ausgewählt aus der Gruppe bestehend aus Halogen, Nitro, Cyano, C₁-C₆-Alkyl, C₁-C₆-Alkoxy, Amino, mono- oder di-C₁-C₆-Alkylamino, Hydroxycarbonyl, C₁-C₆-Alkoxycarbonyl und mono- oder di-C₁-C₆-Alkylaminocarbonyl,
   oder
   - G: für C₃-C₁₀-Cycloalkyl steht, wobei C₃-C₁₀-Cycloalkyl gegebenenfalls substituiert sein kann mit bis zu drei Substituenten ausgewählt aus der Gruppe bestehend aus Halogen, Nitro, Cyano, Hydroxy, C₁-C₆-Alkyl, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   - R¹, R², R³ und R⁴: gleich oder verschieden sind und jeweils für Wasserstoff, Amino, mono- oder di-C₁-C₆-Alkylamino, C₁-C₆-Alkylcarbonylamino, C₆-C₁₀-Aryl oder C₁-C₆-Alkyl, wobei C₁-C₆-Alkyl gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl, stehen,
   und
   wobei C₆-C₁₀-Aryl gegebenenfalls substituiert sein kann mit bis zu drei Substituenten ausgewählt aus der Gruppe bestehend aus Halogen, Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl, mono- oder di-C₁-C₆-Alkylaminocarbonyl und C₁-C₆-Alkyl,
   worin
   C₁-C₆-Alkyl gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   oder
   - R¹ und R² oder R³ und R⁴: bilden zusammen mit dem Kohlenstoffatom, an das sie gebunden sind, einen C₃-C₆-Cycloalkyl-Ring, wobei der C₃-C₆-Cycloalkyl-Ring gegebenenfalls substituiert sein kann mit bis zu drei Substituenten ausgewählt aus der Gruppe bestehend aus Halogen, Hydroxy, C₁-C₆-Alkyl, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   oder
   - R¹ und R³: bilden zusammen mit den Kohlenstoffatomen, an die sie gebunden sind, einen C₃-C₆-Cycloalkyl-Ring, wobei der C₃-C₆-Cycloalkyl-Ring gegebenenfalls substituiert sein kann mit bis zu drei Substituenten ausgewählt aus der Gruppe bestehend aus Halogen, Hydroxy, C₁-C₆-Alkyl, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   - R⁵: für Wasserstoff, Halogen, Hydroxy, C₁-C₆-Alkoxy, Amino, mono- oder di-C₁-C₆-Alkylamino oder C₁-C₆-Alkyl steht, wobei C₁-C₆-Alkoxy, mono- oder di-C₁-C₆-Alkylamino oder C₁-C₆-Alkyl gegebenenfalls substituiert sein können mit bis zu drei Substituenten ausgewählt aus der Gruppe bestehend aus Hydroxy, C₁-C₆-Alkoxy, Amino, mono- oder di-C₁-C₆-Alkylamino, Hydroxycarbonyl, C₁-C₆-Alkoxycarbonyl und mono- oder di-C₁-C₆-Alkylaminocarbonyl,
   - R⁶, R⁷ und R⁸: gleich oder verschieden sind und jeweils für Wasserstoff oder C₁-C₆-Alkyl stehen, wobei C₁-C₆-Alkyl gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   - R⁹ und R¹⁰: gleich oder verschieden sind und jeweils für Wasserstoff, NR¹¹R¹², OR¹³ oder C₁-C₆-Alkyl stehen, wobei C₁-C₆-Alkyl gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   - R¹¹, R¹² und R¹³: gleich oder verschieden sind und jeweils für Wasserstoff oder C₁-C₆-Alkyl stehen, wobei C₁-C₆-Alkyl gegebenenfalls substituiert sein kann mit bis zu drei Substituenten ausgewählt aus der Gruppe bestehend aus Hydroxy, C₁-C₆-Alkoxy, Amino, mono- oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
   sowie deren Tautomere, Stereoisomere, stereoisomere Gemische und deren pharmakologisch verträglichen Salze,
   oder
[B] Verbindungen der Formel (Ib) 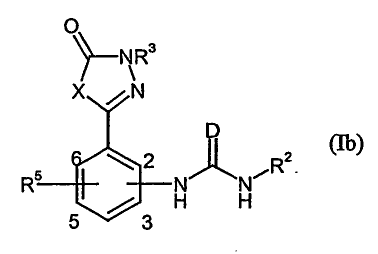 in welcher
   der Rest -NHC(D)NHR² über eine der Positionen 2, 3, 5 oder 6 an den Aromaten gebunden ist,
   - X: für -N(R⁶)- oder eine Gruppe 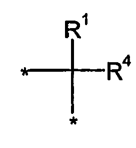 steht,
   - D: für Sauerstoff oder Schwefel steht,
   - R¹: für C₆-C₁₀-Aryl oder C₁-C₆-Alkyl steht, wobei Alkyl gegebenenfalls substituiert sein kann mit bis zu drei Substituenten unabhängig voneinander ausgewählt aus der Gruppe bestehend aus Hydroxy, C₁-C₆-Alkoxy, Amino, C₁-C₆-Alkylamino, C₁-C₆-Alkylcarbonylamino, Hydroxycarbonyl, C₁-C₆-Alkoxycarbonyl und C₁-C₆-Alkylaminocarbonyl,
   und
   wobei Aryl gegebenenfalls substituiert sein kann mit bis zu drei Substituenten unabhängig voneinander ausgewählt aus der Gruppe bestehend aus Halogen, Hydroxy, C₁-C₆-Alkoxy, Amino, C₁-C₆-Alkylamino, C₁-C₆-Alkylcarbonylamino, Hydroxycarbonyl, C₁-C₆-Alkoxycarbonyl, C₁-C₆-Alkylaminocarbonyl und C₁-C₆-Alkyl,
   oder
   - R¹ und R⁴: bilden zusammen mit dem Kohlenstoffatom, an das sie gebunden sind, einen C₃-C₆-Cycloalkyl-Ring, wobei der Cycloalkyl-Ring gegebenenfalls substituiert sein kann mit bis zu drei Substituenten unabhängig voneinander ausgewählt aus der Gruppe bestehend aus Halogen, Hydroxy, C₁-C₆-Alkyl, C₁-C₆-Alkoxy, Amino, C₁-C₆-Alkylamino, C₁-C₆-Alkylcarbonylamino, Hydroxycarbonyl, C₁-C₆-Alkoxycarbonyl und C₁-C₆-Alkylaminocarbonyl,
   - R²: für C₃-C₈-Cycloalkyl oder C₆-C₁₀-Aryl steht, wobei Aryl gegebenenfalls substituiert sein kann mit bis zu drei Substituenten unabhängig voneinander ausgewählt aus der Gruppe bestehend aus Halogen, Hydroxy, Nitro, Cyano, C₁-C₆-Alkoxy, Hydroxycarbonyl, C₁-C₆-Alkoxycarbonyl, Amino, C₁-C₆-Alkylamino, C₁-C₆-Alkylaminocarbonyl und C₁-C₆-Alkyl,
   - R³: für Wasserstoff oder C₁-C₆-Alkyl steht, wobei Alkyl gegebenenfalls substituiert sein kann mit bis zu zwei Substituenten unabhängig voneinander ausgewählt aus der Gruppe bestehend aus C₁-C₆-Alkoxy, Hydroxycarbonyl und C₁-C₆-Alkoxycarbonyl,
   - R⁴: für C₁-C₆-Alkyl steht, wobei Alkyl gegebenenfalls substituiert sein kann mit bis zu drei Substituenten unabhängig voneinander ausgewählt aus der Gruppe bestehend aus Hydroxy, C₆-C₁₀-Aryl, C₁-C₆-Alkoxy, Amino, C₁-C₆-Alkylamino, C₁-C₆-Alkylcarbonylamino, Hydroxy-carbonyl, C₁-C₆-Alkoxycarbonyl und C₁-C₆-Alkylaminocarbonyl,
   oder
   - R⁴: für C₆-C₁₀-Aryl steht, wobei Aryl gegebenenfalls substituiert sein kann mit bis zu drei Substituenten unabhängig voneinander ausgewählt aus der Gruppe bestehend aus Halogen, Hydroxy, C₁-C₆-Alkoxy, Amino, C₁-C₆-Alkylamino, C₁-C₆-Alkylearbonylamino, Hydroxycarbonyl, C₁-C₆-Alkoxycarbonyl, C₁-C₆-Alkylaminocarbonyl und C₁-C₆-Alkyl,
   - R⁵: für Wasserstoff, Halogen, Hydroxy, C₁-C₆-Alkoxy, Amino, C₁-C₆-Alkylamino oder C₁-C₆-Alkyl steht,
   - R⁶: für C₆-C₁₀-Aryl, C₃-C₈-Cycloalkyl oder C₁-C₆-Alkyl steht, wobei Alkyl gegebenenfalls substituiert sein kann mit bis zu zwei Substituenten unabhängig voneinander ausgewählt aus der Gruppe bestehend aus Hydroxy, C₆-C₁₀-Aryl, C₁-C₆-Alkoxy, Amino, C₁-C₆-Alkylamino, Hydroxycarbonyl und C₁-C₆-Alkoxycarbonyl,
   und
   wobei Cycloalkyl gegebenenfalls substituiert sein kann mit bis zu drei Substituenten unabhängig voneinander ausgewählt aus der Gruppe bestehend aus Hydroxy, C₁-C₆-Alkyl, C₆-C₁₀-Aryl, C₁-C₆-Alkoxy, Amino, C₁-C₆-Alkylamino, Hydroxycarbonyl und C₁-C₆-Alkoxycarbonyl,
sowie deren Tautomere, Stereoisomere, stereoisomere Gemische und deren pharmakologisch verträglichen Salze.

Bevorzugt sind Verbindungen der allgemeinen Formel (Ia) worin
A über die Positionen 2, 3, 5 oder 6 an den Aromaten gebunden ist und
- A: für NR⁶ steht,
- E: für NR⁷ steht,
- Y: für NR⁸ steht,
- D und X: für Sauerstoff stehen,
- G: für C₆-C₁₀-Aryl steht, wobei C₆-C₁₀-Aryl gegebenenfalls substituiert sein kann mit bis zu drei Substituenten, die unabhängig voneinander ausgewählt werden aus der Gruppe bestehend aus Halogen, Hydroxy, Cyano und C₁-C₆-Alkyl,
worin
C₁-C₆-Alkyl gegebenenfalls substituiert sein kann mit bis zu drei Substituenten von Halogen,
oder
- G: für C₅-C₆-Heteroaryl steht, wobei C₅-C₆-Heteroaryl gegebenenfalls substituiert sein kann mit bis zu drei Substituenten, die unabhängig voneinander ausgewählt werden aus der Gruppe bestehend aus Halogen und C₁-C₃-Alkyl,
oder
- G: für C₃-C₁₀-Cycloalkyl steht, wobei C₃-C₁₀-Cycloalkyl gegebenenfalls substituiert sein kann mit bis zu drei Substituenten C₁-C₆-Alkyl,
- R¹, R² und R³: gleich oder verschieden sind und jeweils für Wasserstoff oder für C₁-C₃-Alkyl stehen,
- R⁴: für Wasserstoff, C₆-C₁₀-Aryl oder C₁-C₆-Alkyl steht, wobei C₁-C₆-Alkyl gegebenenfalls substituiert sein kann mit bis zu drei Substituenten, die unabhängig voneinander ausgewählt werden aus der Gruppe bestehend aus Hydroxy, C₁-C₆-Alkoxy, Amino, mono-oder di-C₁-C₆-Alkylamino, C₁-C₆-Alkylcarbonylamino, Hydroxycarbonyl, C₁-C₆-Alkoxycarbonyl und mono- oder di-C₁-C₆-Alkylaminocarbonyl,
und
wobei C₆-C₁₀-Aryl gegebenenfalls substituiert sein kann mit bis zu drei Substituenten, die unabhängig voneinander ausgewählt werden aus der Gruppe bestehend aus Halogen, Hydroxy, C₁-C₆-Alkoxy und C₁-C₆-Alkyl,
wobei R¹, R², R³ und R⁴ nicht gleichzeitig Wasserstoff sind,
- R⁵: für Wasserstoff, Halogen, Hydroxy, Amino, mono- oder di-C₁-C₆-Alkylamino oder C₁-C₆-Alkyl steht, wobei C₁-C₆-Alkyl gegebenenfalls substituiert sein kann mit bis zu drei Substituenten, die unabhängig voneinander ausgewählt werden aus der Gruppe bestehend aus Hydroxy, C₁-C₆-Alkoxy, Amino, mono- oder di-C₁-C₆-Alkylamino, Hydroxycarbonyl, C₁-C₆-Alkoxycarbonyl und mono- oder di-C₁-C₆-Alkylaminocarbonyl,
- R⁶, R⁷ und R⁸: für Wasserstoff stehen,
oder
Verbindungen der allgemeinen Formel (Ib) worin
der Rest -NHC(D)NHR² über eine der Positionen 2, 3, 5 oder 6 an den Aromaten gebunden ist,
- X: für -N(R⁶)- oder eine Gruppe 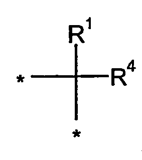 steht,
- D: für Sauerstoff steht,
- R¹: für C₁-C₆-Alkyl steht, wobei Alkyl gegebenenfalls substituiert sein kann mit bis zu drei Substituenten unabhängig voneinander ausgewählt aus der Gruppe bestehend aus Hydroxy, C₁-C₆-Alkoxy, Amino, C₁-C₆-Alkylamino, C₁-C₆-Alkylcarbonylamino, Hydroxycarbonyl, C₁-C₆-Alkoxycarbonyl und C₁-C₆-Alkylaminocarbonyl,
oder
- R¹ und R⁴: bilden zusammen mit dem Kohlenstoffatom, an das sie gebunden sind, einen C₅-C₆-Cycloalkyl-Ring, wobei der Cycloalkyl-Ring gegebenenfalls substituiert sein kann mit bis zu drei Substituenten unabhängig voneinander ausgewählt aus der Gruppe bestehend aus Halogen, Hydroxy, C₁-C₆-Alkyl, C₁-C₆-Alkoxy, Amino, C₁-C₆-Alkylamino, C₁-C₆-Alkylcarbonylamino, Hydroxycarbonyl, C₁-C₆-Alkoxycarbonyl und C₁-C₆-Alkylaminocarbonyl,
- R²: für C₆-C₁₀-Aryl steht, wobei Aryl gegebenenfalls substituiert sein kann mit bis zu drei Substituenten unabhängig voneinander ausgewählt aus der Gruppe bestehend aus Halogen oder C₁-C₆-Alkyl,
- R³: für Wasserstoff oder C₁-C₆-Alkyl steht, wobei Alkyl gegebenenfalls substituiert sein kann mit bis zu zwei Substituenten unabhängig voneinander ausgewählt aus der Gruppe bestehend aus C₁-C₆-Alkoxy, Hydroxycarbonyl und C₁-C₆-Alkoxycarbonyl,
- R⁴: für C₁-C₆-Alkyl steht, wobei Alkyl gegebenenfalls substituiert sein kann mit bis zu drei Substituenten unabhängig voneinander ausgewählt aus der Gruppe bestehend aus Hydroxy, Phenyl, C₁-C₆-Alkoxy, Amino, C₁-C₆-Alkylamino, C₁-C₆-Alkylcarbonylamino, Hydroxycarbonyl, C₁-C₆-Alkoxycarbonyl und C₁-C₆-Alkylaminocarbonyl,
- R⁵: für Wasserstoff, Halogen, Hydroxy, C₁-C₆-Alkoxy, Amino, C₁-C₆-Alkylamino oder C₁-C₆-Alkyl steht,
- R⁶: für C₃-C₈-Cycloalkyl oder C₁-C₆-Alkyl steht, wobei Alkyl gegebenenfalls substituiert sein kann mit bis zu zwei Substituenten unabhängig voneinander ausgewählt aus der Gruppe bestehend aus Hydroxy, C₆-C₁₀-Aryl, C₁-C₆-Alkoxy, Amino, C₁-C₆-Alkylamino, Hydroxycarbonyl und C₁-C₆-Alkoxycarbonyl,
und
wobei Cycloalkyl gegebenenfalls substituiert sein kann mit bis zu drei Substituenten unabhängig voneinander ausgewählt aus der Gruppe bestehend aus C₁-C₆-Alkyl und C₁-C₆-Alkoxy.

Besonders bevorzugt sind
N-(2,4-Difluorphenyl)-N'-[3-(4,4-dimethyl-6-oxo-1,4,5,6-tetrahydro-3-pyridazinyl)-phenyl]harnstoff 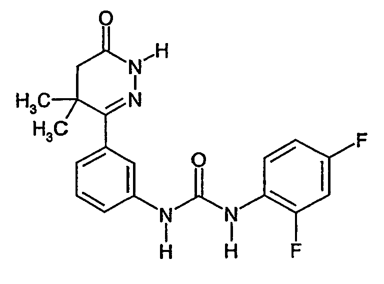 N-(2,5-Difluorphenyl)-N'-[3-(4,4-dimethyl-6-oxo-1,4,5,6-tetrahydro-3-pyridazinyl)-4-hydroxyphenyl]harnstoff 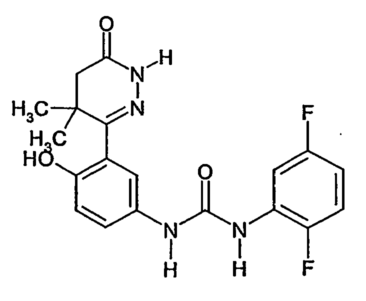 N-[4-({[(3-Chlor-4-fluorphenyl)amino]carbonyl}amino)phenyl]acetamid 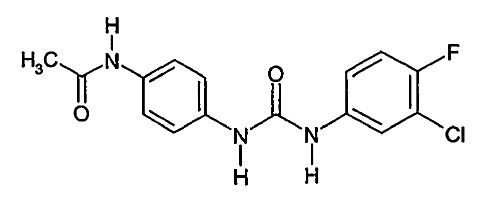 N-[4-({[(3-Chlor-4-fluorphenyl)amino]carbonyl}amino)phenyl]pentanamid 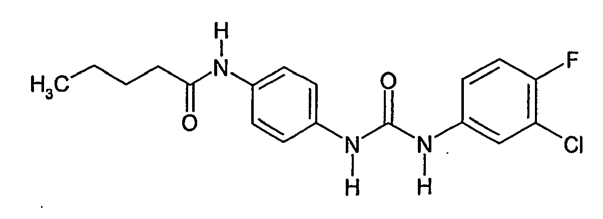 N-[3-({[(3-CMor-4-fluorphenyl)amino]carbonyl}amino)phenyl]-1-butansulfonamid 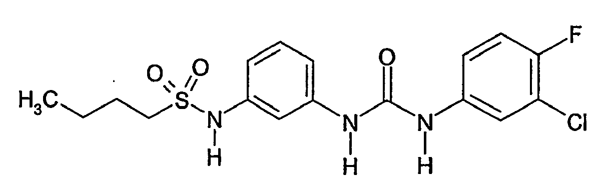 1-(3-Chlor-4-fluor-phenyl)-3-[3-(4-isopropyl-5-oxo-4,5-dihydro-1H-[1,2,4]triazol-3-yl)-phenyl]harnstoff 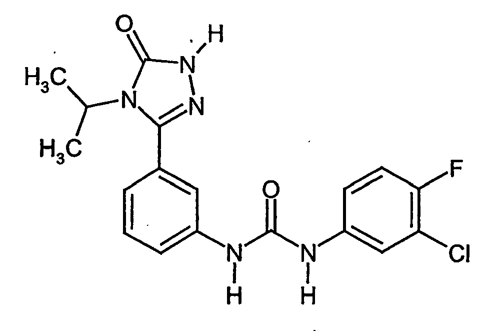 1-(3-Chlor-4-fluor-phenyl)-3-[3-(4-cyclohexyl-5-oxo-4,5-dihydro-1H-[1,2,4]hiazol-3-yl)-phenyl]-harnstoff 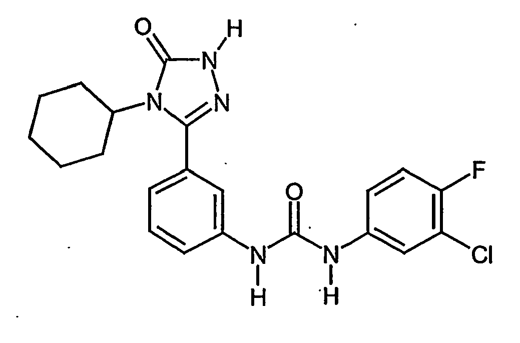 N-(4-Chlor-2-methylphenyl)-N¹-[3-4,4-dimethyl-5-oxo-4,5-dihydro-1*H*-pyrazol-3-yl)phenyl]-harnstoff

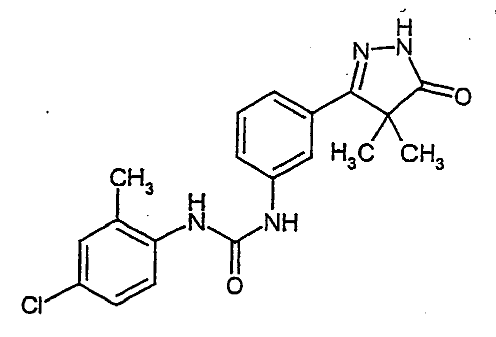

Die Verbindungen können in Abhängigkeit von ihrer Struktur in stereoisomeren Formen (Enantiomere, Diastereomere) existieren. Die Erfindung betrifft deshalb die Enantiomeren oder Diastereomeren and ihre jeweiligen Mischungen. Aus solchen Mischungen von Enantiomeren und/oder Diastereomeren lassen sich die stereoisomer einheitlichen Bestandteile in bekannter Weise isolieren.

Umfasst sind in Abhängigkeit von der Struktur der Verbindungen auch Tautomere der Verbindungen.

Als Salze sind physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren and Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure and Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabietylamin, Arginin, Lysin, Ethylendiamin and Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylaminocarbonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.
Alkoxy steht beispielhaft und vorzugsweise für Methoxy, Ethoxy, n-Propoxy, Isopropoxy, tert.-Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino,
Alkylaminocarbonyl steht für einen Alkylaminocarbonykest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert.-Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-t-Butyl-*N*-methylaminocarbonyl, *N*-Ethyl-*N*-n-pentylamino-carbonyl und *N*-n-Hexyl-*N*-methylaminocarbonyl.
Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.
Cycloalkyl steht für eine Cycloalkylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl, Cycloheptyl und Adamantyl.
Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.
5- bis 10-gliedriges Heteroaryl ("C₅-C₁₀-Heteroaryl") steht im Rahmen der Erfindung für 5- bis 10-gliedrige, Heteroatome enthaltende aromatische Ringe mit wenigstens einem aromatischen Ring, die 1 bis 4 Heteroatome enthalten können, die ausgewählt werden aus O, S und N. Heteroaryl kann seinerseits noch über C oder N substituiert sein. Beispielsweise seien genannt: Pyridyl, Furyl, Thienyl, Pyrrolyl, Imidazolyl, Pyrazolyl, Pyrazinyl, Pyrimidinyl, Pyridazinyl, Indolicenyl, Indolyl, Benzo[b]thienyl, Benzo[b]furyl, Indazolyl, Chinolyl, Isochinolyl, Naphthyridinyl, Chinazolinyl, etc.
Ein 5- bis 7-gliedriger gesättigter oder teilweise ungesättigter Heterocyclus ("C₅-C₇-Heterocyclyl") mit bis zu 3 Heteroatomen aus der Reihe S, N und/oder O steht im Rahmen der Erfindung im allgemeinen für einen mono- oder polycyclischen, vorzugsweise mono- oder bicyclischen Heterocyclus, der eine oder mehrere Doppelbindungen enthalten kann und der über ein Ringkohlenstoffatom oder ein Ringstickstoffatom verknüpft ist. Heterocyclyl kann seinerseits noch über C oder N substituiert sein. Beispielsweise seien genannt: Tetrahydrofuryl, Pyrrolidin-2-yl, Pyrrolidin-3-yl, Pyrrolinyl, Piperidinyl, Dihydropyridinyl, Piperazinyl, Morpholinyl, Azepinyl, Diazepinyl. Bevorzugt sind Piperidinyl, Morpholinyl und Pyrrolidinyl.
Halogen steht für Fluor, Chlor, Brom und Jod, bevorzugt Fluor und Chlor.

Die Verbindungen der Formel (Ia) und (Ib) sind bekannt oder lassen sich nach den folgenden Verfahren herstellen.

Bei Verfahren
[A] setzt man Verbindungen der allgemeinen Formel (IIa), 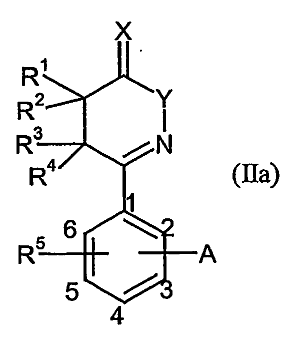 in welcher
   A über die Positionen 2, 3, 5 oder 6 an den Aromaten gebunden ist und
   R¹, R², R³, R⁴, R⁵, A, X und Y die oben angegebene Bedeutung aufweisen,
   mit Verbindungen der allgemeinen Formel (IIIa),

   D=C=N-G (IIIa)

   in welcher
   D und G die oben angegebene Bedeutung aufweisen,
   zu Verbindungen der allgemeinen Formel (Iaa), 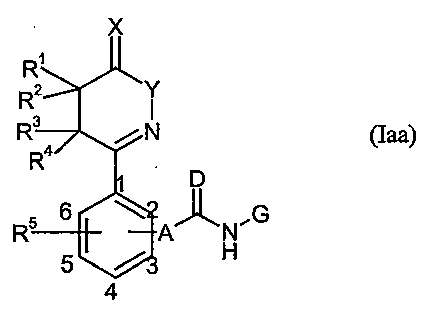 in welcher
   A über die Positionen 2, 3, 5 oder 6 an den Aromaten gebunden ist, und
   R¹, R², R³, R⁴, R⁵, A, D, G, X und Y die oben angegebene Bedeutung aufweisen,
   in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, als Lösungsmittel sind bevorzugt Tetrahydrofuran oder Methylenchlorid, gegebenenfalls in Gegenwart einer Base, wie beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin, bevorzugt in einem Temperaturbereich von Raumtemperatur bis zum Rückfluss der Lösungsmittel bei Normaldruck, um.
   Die Verbindungen der allgemeinen Formel (IIa) werden im Folgenden als (IIaa), (IIba) und (IIca) dargestellt.
   Die Verbindungen der allgemeinen Formel (IIIa) sind bekannt oder lassen sich nach bekannten Verfahren aus den entsprechenden Edukten synthetisieren.

Bei Verfahren
[B] setzt man Verbindungen der allgemeinen Formel (IIa) mit Verbindungen der allgemeinen Formel (IVa), 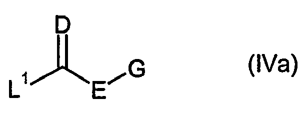 in welcher
   - D, E und G: die oben angegebene Bedeutung aufweisen, und
   - L¹: für p-Nitrophenyl oder Halogen, bevorzugt Brom oder Chlor, steht,
   zu Verbindungen der allgemeinen Formel (Iaa), 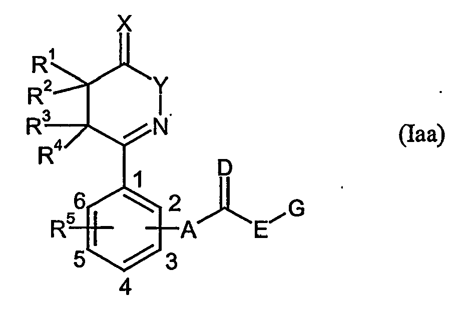 in welcher
   A über die Positionen 2, 3, 5 oder 6 an den Aromaten gebunden ist, und
   R¹, R², R³, R⁴, R⁵, A, D, E, G, X und Y die oben angegebene Bedeutung aufweisen, in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Acetonitril oder Pyridin, als Lösungsmittel sind bevorzugt Tetrahydrofuran oder Methylenchlorid, gegebenenfalls in Gegenwart einer Base, wie beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin, bevorzugt in einem Temperaturbereich von Raumtemperatur bis zum Rückfluss der Lösungsmittel bei Normaldruck, um.
   Die Verbindungen der allgemeinen Formel (IVa) sind bekannt oder lassen sich nach bekannten Verfahren aus den entsprechenden Edukten synthetisieren.

Bei Verfahren
[C] setzt man Verbindungen der allgemeinen Formel (Va), 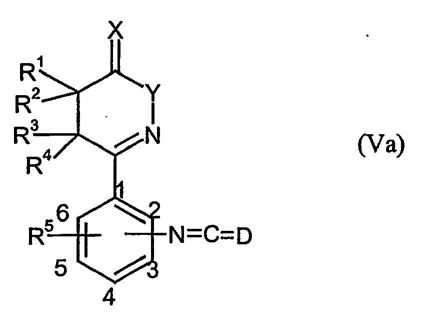 in welcher
   -NCD über die Positionen 2, 3, 5 oder 6 an den Aromaten gebunden ist, und
   R¹, R², R³, R⁴, R⁵, D, X und Y die oben angegebene Bedeutung aufweisen,
   mit Verbindungen der allgemeinen Formel (VIa),

   H-M-G (VIa)

   in welcher
   - G: die oben angegebene Bedeutung aufweist, und
   - M: für Sauerstoff oder NR⁷ steht,
   worin
   R⁷ die oben angegebene Bedeutung aufweist,
   zu Verbindungen der allgemeinen Formel (Iba), 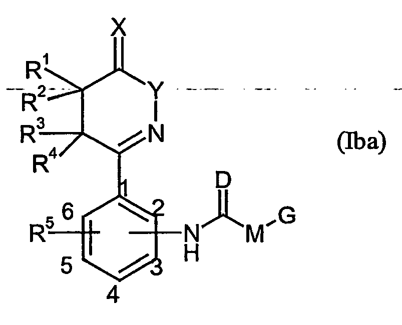 in welcher
   -NH-C(D)-M-G über die Positionen 2,3, 5 oder 6 an den Aromaten gebunden ist, und
   R¹, R², R³, R⁴, R⁵, D, G, M, X und Y die oben angegebene Bedeutung aufweisen,
   in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, als Lösungsmittel sind bevorzugt Tetrahydrofuran oder Methylenchlorid, gegebenenfalls in Gegenwart einer Base, wie beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin, bevorzugt in einem Temperaturbereich von Raumtemperatur bis zum Rückfluss der Lösungsmittel bei Normaldruck, um.

Die Verbindungen der allgemeinen Formel (VIa) sind bekannt oder lassen sich nach bekannten Verfahren aus den entsprechenden Edukten synthetisieren.

Zur Herstellung der Verbindungen der allgemeinen Formel (IIaa), 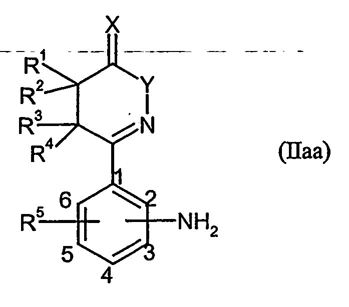 in welcher
NH₂ über die Positionen 2, 3, 5 oder 6 an den Aromaten gebunden ist und
R¹, R², R³, R⁴, R⁵, X und Y die oben angegebene Bedeutung aufweisen,
setzt man Verbindungen der allgemeinen Formel (VIIa), 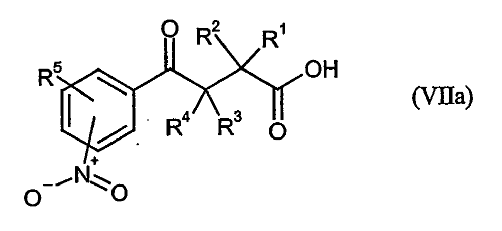 in welcher
NO₂ über die Positionen 2, 3, 5 oder 6 an den Aromaten gebunden ist und
R¹, R², R³, R⁴ und R⁵ die oben angegebene Bedeutung aufweisen,
im Falle, wenn für X Sauerstoff steht,
zunächst mit Hydrazin, Hydroxylamin oder einer Verbindung der allgemeinen Formel (VIIIa), 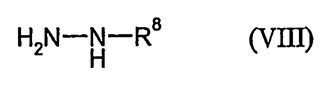 in welcher
- R⁸: die oben angegebene Bedeutung aufweist,
um und reduziert anschließend die Nitrogruppe zur Aminogruppe. Diese beiden Reaktionen können in ein oder zwei Reaktionsschritten stattfinden.

Bei einem einstufigen Verfahren wird mit Hydrazin und mit Palladium auf Kohle gleichzeitig in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylacetamid, Acetonitril oder Pyridin, als Lösungsmittel sind bevorzugt Ethanol oder iso-Propanol, bevorzugt in einem Temperaturbereich von Raumtemperatur bis zum Rückfluss der Lösungsmittel bei Normaldruck, umgesetzt.

Bei einem zweistufigen Verfahren wird zunächst mit Hydrazin, Hydroxylamin oder einer Verbindung der allgemeinen Formel (VIIIa) in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Ethanol oder iso-Propanol, bevorzugt in einem Temperaturbereich von Raumtemperatur bis zum Rückfluss der Lösungsmittel bei Normaldruck, umgesetzt.

In der zweiten Stufe wird mit Wasserstoffdonoren, bevorzugt Hydrazin oder Wasserstoff und mit Palladium auf Kohle, oder mit Zinndichlorid in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Ethylacetat, Dimethylformamid, Dimethylacetamid, Acetonitril oder Pyridin, als Lösungsmittel sind bevorzugt Ethanol, iso-Propanol oder im Falle von Zinndichlorid in Dimethylformamid, bevorzugt in einem Temperaturbereich von Raumtemperatur bis zum Rückfluss der Lösungsmittel bei Normaldruck bis 3 bar, umgesetzt.

Im Falle, wenn für X Schwefel steht,
wird zunächst mit Hydrazin, Hydroxylamin oder einer Verbindung der allgemeinen Formel (VIIIa) umgesetzt, dann mit Lawesson-Reagenz der Sauerstoff gegen Schwefel ausgetauscht und anschließend die Nitrogruppe zur Aminogruppe reduziert.

In der ersten Stufe wird zunächst mit Hydrazin, Hydroxylamin oder einer Verbindung der allgemeinen Formel (VIIIa) in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Ethanol oder iso-Propanol, bevorzugt in einem Temperaturbereich von Raumtemperatur bis zum Rückfluss der Lösungsmittel bei Normaldruck, umgesetzt.

In der zweiten Stufe wird mit Lawesson-Reagenz in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, 1,2-Dimethoxyethan, Dimethylsulfoxid oder Pyridin, bevorzugt sind Toluol, Xylol oder Dioxan, bevorzugt in einem Temperaturbereich von Raumtemperatur bis zum Rückfluss der Lösungsmittel bei Normaldruck, durchgeführt.

In der dritten Stufe wird mit Wasserstoffdonoren, bevorzugt Hydrazin oder Wasserstoff und mit Palladium auf Kohle, oder mit Zinndichlorid in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Ethanol, iso-Propanol oder im Falle von Zinndichlorid in Dimethylformamid, bevorzugt in einem Temperaturbereich von Raumtemperatur bis zum Rückfluss der Lösungsmittel bei Normaldruck bis 3 bar, umgesetzt.

Verbindungen der allgemeinen Formel (VIIa) können in zwei verschiedenen Formen vorliegen. Im Verlaufe der Beschreibung der Verfahren wird nur die Offenkettige-Form gezeichnet.

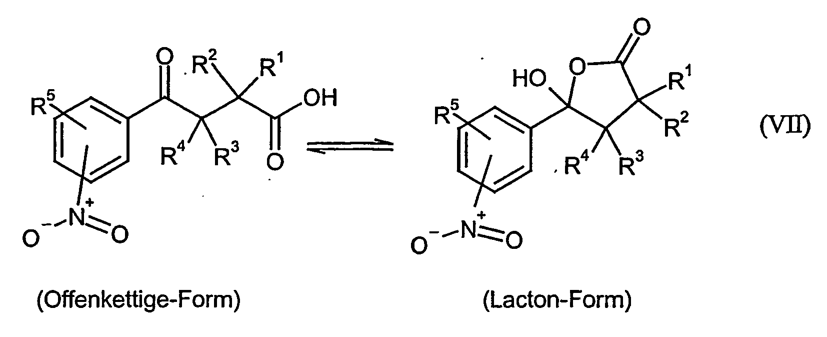

Zur Herstellung der Verbindungen der allgemeinen Formel (IIba), 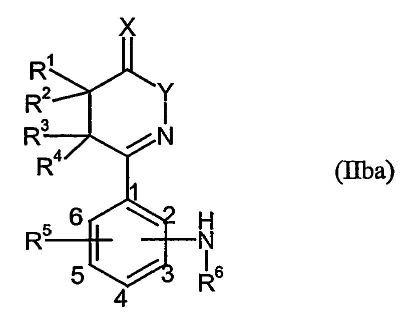 in welcher
NHR⁶ über die Positionen 2, 3, 5 oder 6 an den Aromaten gebunden ist und
R¹, R², R³, R⁴, R⁵, R⁶, X und Y die oben angegebene Bedeutung aufweisen,
setzt man Verbindungen der allgemeinen Formel (IIaa) mit Verbindungen der allgemeinen Formel (IXa),

L²-R⁶ (IXa)

in welcher
- R⁶: die oben angegebene Bedeutung aufweist, und
- L²: für Halogen, bevorzugt Brom oder Iod, steht,
in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Tetradydrofuran oder Diethylether, gegebenenfalls in Gegenwart einer Base, wie beispielsweise Alkalihydroxide wie Natrium- oder Kaliumhydroxid, oder Alkalicarbonate wie Cäsiumcarbonat, Natrium- oder Kaliumcarbonat, oder Amide wie Natriumamid, Lithium-bis-(trimethylsilyl)amid, Lithiumdiisopropylamid, oder metallorganische Verbindungen wie Butyllithium oder Phenyllithium, oder andere Basen wie Natriumhydrid, DBU, Triethylamin oder Diisopropylethylamin, bevorzugt Diisopropylethylamin, Kalium-tert.-butylat oder DBU, bevorzugt in einem Temperaturbereich von Raumtemperatur bis zum Rückfluss der Lösungsmittel bei Normaldruck, umgesetzt.

Die Verbindungen der allgemeinen Formel (IXa) sind bekannt oder lassen sich nach bekannten Verfahren aus den entsprechenden Edukten synthetisieren.

Zur Herstellung der Verbindungen der allgemeinen Formel (IIca), 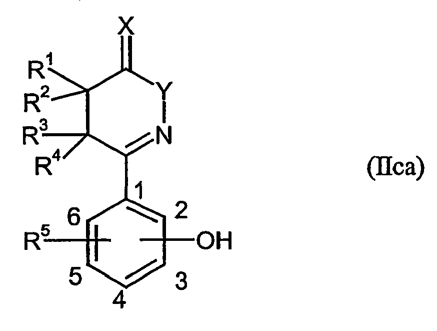 in welcher
OH über die Positionen 2, 3, 5 oder 6 an den Aromaten gebunden ist und
R¹, R², R³, R⁴, R⁵, X und Y die oben angegebene Bedeutung aufweisen,
stellt man aus Verbindungen der allgemeinen Formel (IIaa) zunächst die Diazoniumverbindungen nach den dem Fachmann bekannten Methoden her und verkocht diese anschließend zu den Phenolen (vgl. Organikum, 17. Auflage, VEB Deutscher Verlag der Wissenschaften, Berlin, S. 543).

Zur Herstellung der Verbindungen der allgemeinen Formel (Va) werden Verbindungen der allgemeinen Formel (IIaa)
mit Chlorameisensäuretrichlormethylester
in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Acetonitril oder Pyridin umgesetzt. Als Lösungsmittel sind bevorzugt Tetrahydrofuran oder Dichlormethan, gegebenenfalls in Gegenwart einer Base, wie beispielsweise 1,8-Bis-(dimethylamino)naphthalin, DBU, Triethylamin oder Diisopropylethylamin, bevorzugt 1,8-Bis-(dimethylamino)naphthalin, bevorzugt in einem Temperaturbereich von Raumtemperatur bis zum Rückfluss der Lösungsmittel bei Normaldruck.

Zur Herstellung der Verbindungen der allgemeinen Formel (VIIa) setzt man Verbindungen der allgemeinen Formel (Xa), 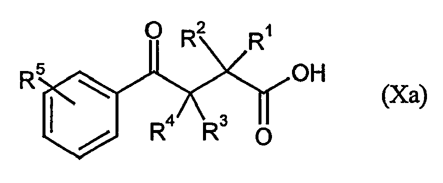 in welcher
R¹, R², R³, R⁴ und R⁵ die oben angegebene Bedeutung aufweisen,
mit rauchender Salpetersäure, konzentrierter Salpetersäure oder Nitriersäure bevorzugt in einem Temperaturbereich von -30°C bis 0°C bei Normaldruck, um.

Verbindungen der allgemeinen Formel (Xa) können in zwei verschiedenen Formen vorliegen. Im Verlaufe der Beschreibung der Verfahren wird nur die Offenkettige-Form gezeichnet.

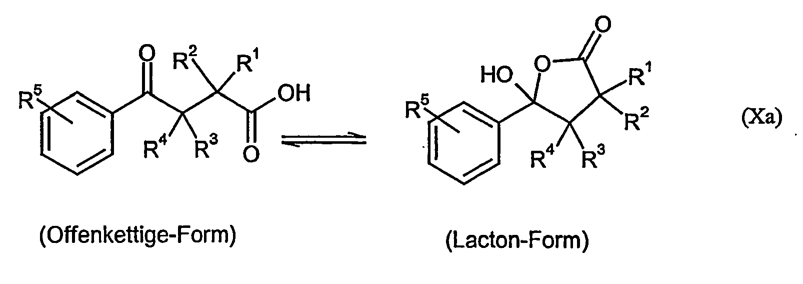

Zur Herstellung der Verbindungen der allgemeinen Formel (Xa) werden Verbindungen der allgemeinen Formel (XIa), 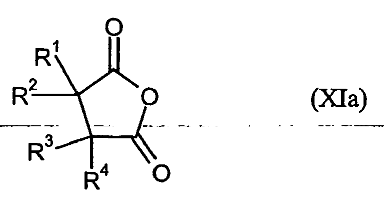 in welcher
R¹, R², R³ und R⁴ die oben angegebene Bedeutung aufweisen,
mit Verbindungen der allgemeinen Formel (XIIa), 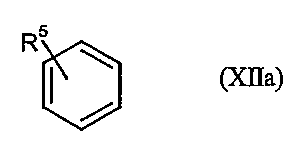 in welcher
R⁵ die oben angegebene Bedeutung aufweist,
mit Lewissäuren, bevorzugt Aluminiumtrichlorid,
in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Nitrobenzol, Hexan, Cyclohexan oder Erdölfraktionen, oder anderen Lösungsmittel wie Ethylacetat, Aceton, Dimethylformamid, Dimethylacetamid, 2-Butanon, Dimethylsulfoxid, Acetonitril oder Pyridin (als Lösungsmittel ist bevorzugt 1,2-Dichlorethan) bevorzugt in einem Temperaturbereich von -20 °C bis Raumtemperatur bei Normaldruck, umgesetzt.

Die Verbindungen der allgemeinen Formel (XIa) und (XIIa) sind bekannt oder lassen sich nach bekannten Verfahren aus den entsprechenden Edukten synthetisieren.

Als alternativen Syntheseweg setzt man zur Herstellung der Verbindungen der allgemeinen Formel (Xaa), welches Verbindungen der allgemeinen Formel (Xa) sind, in denen
- R²: für Wasserstoff steht,

Verbindungen der allgemeinen Formel (XIIIaa), 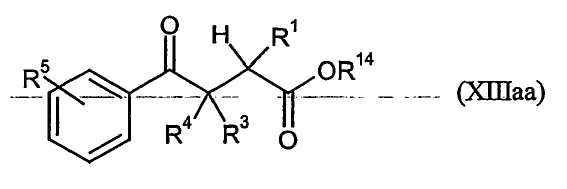 in welcher
- R¹, R³, R⁴ und R⁵: die oben angegebene Bedeutung aufweisen, und
- R¹⁴: für (C₁-C₆)-Alkyl, bevorzugt Methyl und Ethyl, steht,
mit Basen, wie beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt Natriumhydroxid, in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemischen von Lösungsmitteln (als Lösungsmittel sind bevorzugt Tetrahydrofuran und/oder Methanol) bevorzugt in einem Temperaturbereich von 0°C bis Raumtemperatur bei Normaldruck, um:

Die Verbindungen der allgemeinen Formel (Xa) können auch analog zu dem für Verfahren der Verbindungen der allgemeinen Formel (Xaa) beschriebenen Syntheseweg aus den Verbindungen der allgemeinen Formel (XIIIa) hergestellt werden.

Zur Herstellung der Verbindungen der allgemeinen Formel (XIIIaa) setzt man Verbindungen der allgemeinen Formel (XIVa), 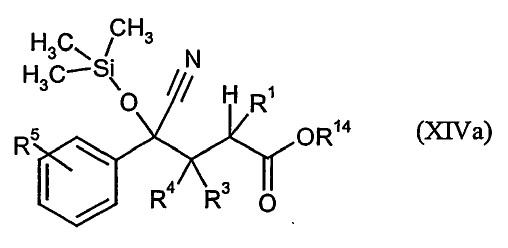 in welcher
R¹, R³, R⁴, R⁵ und R¹⁴ die oben angegebene Bedeutung aufweisen,
mit Tetrabutylammoniumfluorid
in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 2-Butanon, Dimethylsulfoxid, Acetonitril oder Pyridin (als Lösungsmittel ist bevorzugt Tetrahydrofuran) bevorzugt in einem Temperaturbereich von 0°C bis Raumtemperatur bei Normaldruck um.

Zur Herstellung der Verbindungen der allgemeinen Formel (XIVa) setzt man Verbindungen der allgemeinen Formel (XVa), 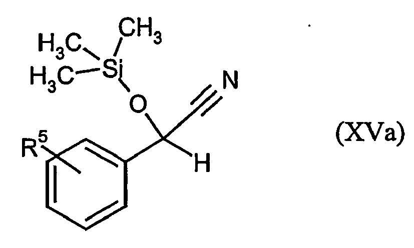 in welcher
R⁵ die oben angegebene Bedeutung aufweist,
mit Verbindungen der allgemeinen Formel (XVIa), 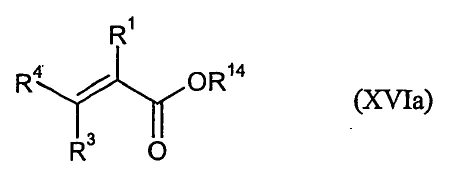 in welcher
R¹, R³, R⁴ und R¹⁴ die oben angegebene Bedeutung aufweisen,
in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Ethylbenzol, Xylol, Toluol, Hexan, Heptan, Cyclohexan oder Erdölfraktionen, oder andere Lösungsmittel wie Dimethylformamid, Dimethylacetamid, Acetonitril oder Pyridin, oder Gemische der Lösungsmittel, als Lösungsmittel sind bevorzugt Diethylether, Tetrahydrofuran, Heptan und/oder Ethylbenzol, gegebenenfalls in Gegenwart einer Base, wie beispielsweise Alkalihydroxide wie Natrium- oder Kaliumhydroxid, oder Alkalicarbonate wie Cäsiumcarbonat, Natrium- oder Kaliumcarbonat, oder Natrium- oder Kaliummethanolat, oder Natrium- oder Kaliumethanolat oder Kalium-tert.butylat, oder Amide wie Natriumamid, Lithium-bis-(trimethylsilyl)amid, Lithiumdiisopropylamid, oder metallorganische Verbindungen wie Butyllithium oder Phenyllithium, oder andere Basen wie Natriumhydrid, DBU, Triethylamin oder Diisopropylethylamin, bevorzugt Lithiumdiisopropylamid, bevorzugt in einem Temperaturbereich von -78°C bis Raumtemperatur bei Normaldruck, um.

Die Verbindungen der allgemeinen Formel (XVIa) sind bekannt oder lassen sich nach bekannten Verfahren aus den entsprechenden Edukten synthetisieren.

Zur Herstellung der Verbindungen der allgemeinen Formel (XVa) setzt man Verbindungen der allgemeinen Formel (XVIIa), 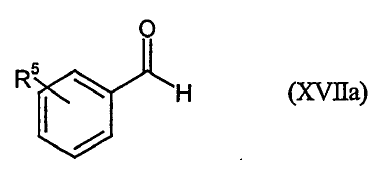 in welcher
R⁵ die oben angegebene Bedeutung aufweist,
mit Trimethylsilylcyanid und Zinkiodid
gegebenenfalls in inerten Lösungsmitteln, hierzu gehören Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 2-Butanon, Dimethylsulfoxid, Acetonitril oder Pyridin (als Lösungsmittel bevorzugt ist Tetrahydrofuran) bevorzugt in einem Temperaturbereich von Raumtemperatur bis 100°C bei Normaldruck, um.

Die Verbindungen der allgemeinen Formel (XVIIa) sind bekannt oder lassen sich nach bekannten Verfahren aus den entsprechenden Edukten synthetisieren.

Zur Herstellung der Verbindungen der allgemeinen Formel (XIIIa), 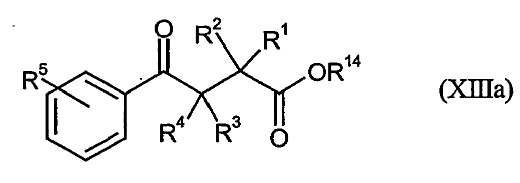 in welcher
R¹, R², R³, R⁴, R⁵ und R¹⁴ die oben angegebene Bedeutung aufweisen,
setzt man Verbindungen der allgemeinen Formel (XVIIIa), 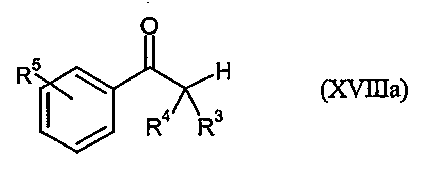 in welcher
R³, R⁴ und R⁵ die oben angegebene Bedeutung aufweisen,
mit Verbindungen der allgemeinen Formel (XIXa), 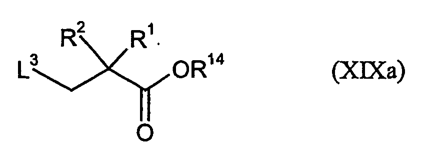 in welcher
- R¹, R² und R¹⁴: die oben angegebene Bedeutung aufweisen, und
- L³: für Halogen, bevorzugt Brom oder Iod, steht,
in inerten Lösungsmitteln, hierzu gehören Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Ethylbenzol, Xylol, Toluol, als Lösungsmittel sind bevorzugt Tetrahydrofuran oder Toluol, gegebenenfalls in Gegenwart einer Base, wie beispielsweise Amide wie Natriumamid, Lithiumhexamethyldisilazid, Kaliumhexamethyldisilazid, Lithiumdiisopropylamid, oder andere Basen wie Natriumhydrid, DBU oder Diisopropylethylamin, bevorzugt Natriumamid, Lithiumhexamethyldisilazid, Kaliumhexamethyldisilazid oder Lithiumdiisopropylamid, bevorzugt in einem Temperaturbereich von -78°C bis Raumtemperatur bei Normaldruck, um.

Die Verbindungen der allgemeinen Formel (XVMA) und (XIXa) sind bekannt oder lassen sich nach bekannten Verfahren aus den entsprechenden Edukten synthetisieren (für (XVIIIa) vgl. M.R. Schneider, H. Ball, J. Med. Chem. 1986, 29, 75-79; Robl, et al., Synthesis 1991, 56; J. Org. Chem. 1996, 61, 607).

In einem alternativen Syntheseweg zur Herstellung der Verbindungen der allgemeinen Formel (IIaaa), welches Verbindungen der allgemeinen Formel (IIaa) sind, in denen
R¹ und R² für Wasserstoff stehen,
setzt man Verbindungen der allgemeinen Formel (XXa), 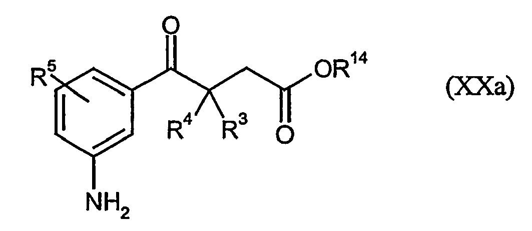 in welcher
R³, R⁴, R⁵ und R¹⁴ die oben angegebene Bedeutung aufweisen,
mit Hydrazin um. Die Umsetzung erfolgt analog der ersten Stufe des zweistufigen Verfahrens, das zur Herstellung der Verbindungen der allgemeinen Formel (IIaa) beschieben ist.

Zur Herstellung der Verbindungen der allgemeinen Formel (XXa), setzt man Verbindungen der allgemeinen Formel (XXIa), 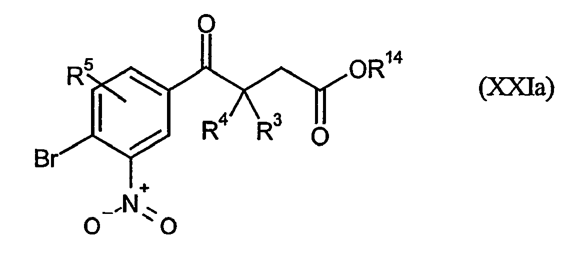 in welcher
R³, R⁴, R⁵ und R¹⁴ die oben angegebene Bedeutung aufweisen,
mit Reduktionsmitteln um. Die Umsetzung erfolgt analog der zweiten Stufe des zweistufigen Verfahrens, das zur Herstellung der Verbindungen der allgemeinen Formel (IIaa) beschieben ist.

Zur Herstellung der Verbindungen der allgemeinen Formel (XXIa), setzt man Verbindungen der allgemeinen Formel (XXIIa), 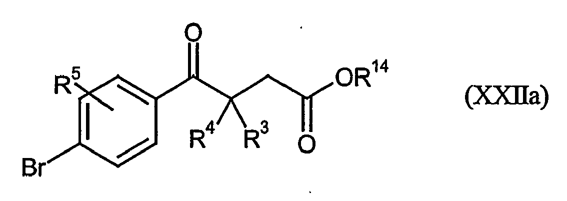 in welcher
R³, R⁴, R⁵ und R¹⁴ die oben angegebene Bedeutung aufweisen,
mit rauchender Salpetersäure, konzentrierter Salpetersäure oder Nitriersäure analog des Verfahrens, das zur Herstellung der Verbindungen der allgemeinen Formel (VIIa) beschieben ist, um.

Die Verbindungen der allgemeinen Formel (XXIIa) lassen sich nach dem für die Verbindungen der allgemeinen Formel (XIIIa) beschriebenen Verfahren aus den entsprechenden Edukten synthetisieren.

In einem alternativen Syntheseweg zur Herstellung der Verbindungen der allgemeinen Formel (XXa), setzt man Verbindungen der allgemeinen Formel (XXIIIa), 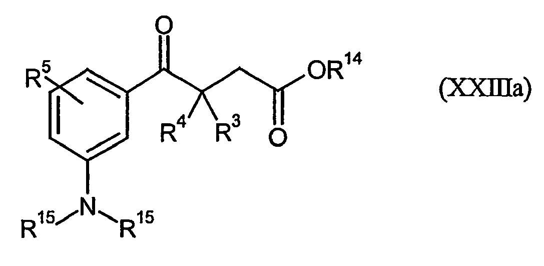 in welcher
R³, R⁴, R⁵ und R¹⁴ die oben angegebene Bedeutung aufweisen,
und
- R¹⁵: für Allyl oder Benzyl steht,
im Falle von Benzyl mit Reduktionsmitteln um. Die Umsetzung erfolgt analog der zweiten Stufe des zweistufigen Verfahrens, das zur Herstellung der Verbindungen der allgemeinen Formel (IIaa) beschieben ist.

Im Falle von Allyl wird ein Verfahren mit Tetrakistriphenylphosphinpalladium und N,N-Dimethylbarbitursäure verwendet, vergl. F. Garro-Helion, A. Merzouk, F. Guibe, J. Org. Chem. 1993, 58, 6109-6113.

Die Verbindungen der allgemeinen Formel (XXIIIa) lassen sich nach dem für die Verbindungen der allgemeinen Formel (XIIIa) beschreibenen Verfahren aus den entsprechenden Edukten synthetisieren.

Die oben beschriebenen Verfahren können durch die folgenden Formelschemata beispielhaft erläutert werden:

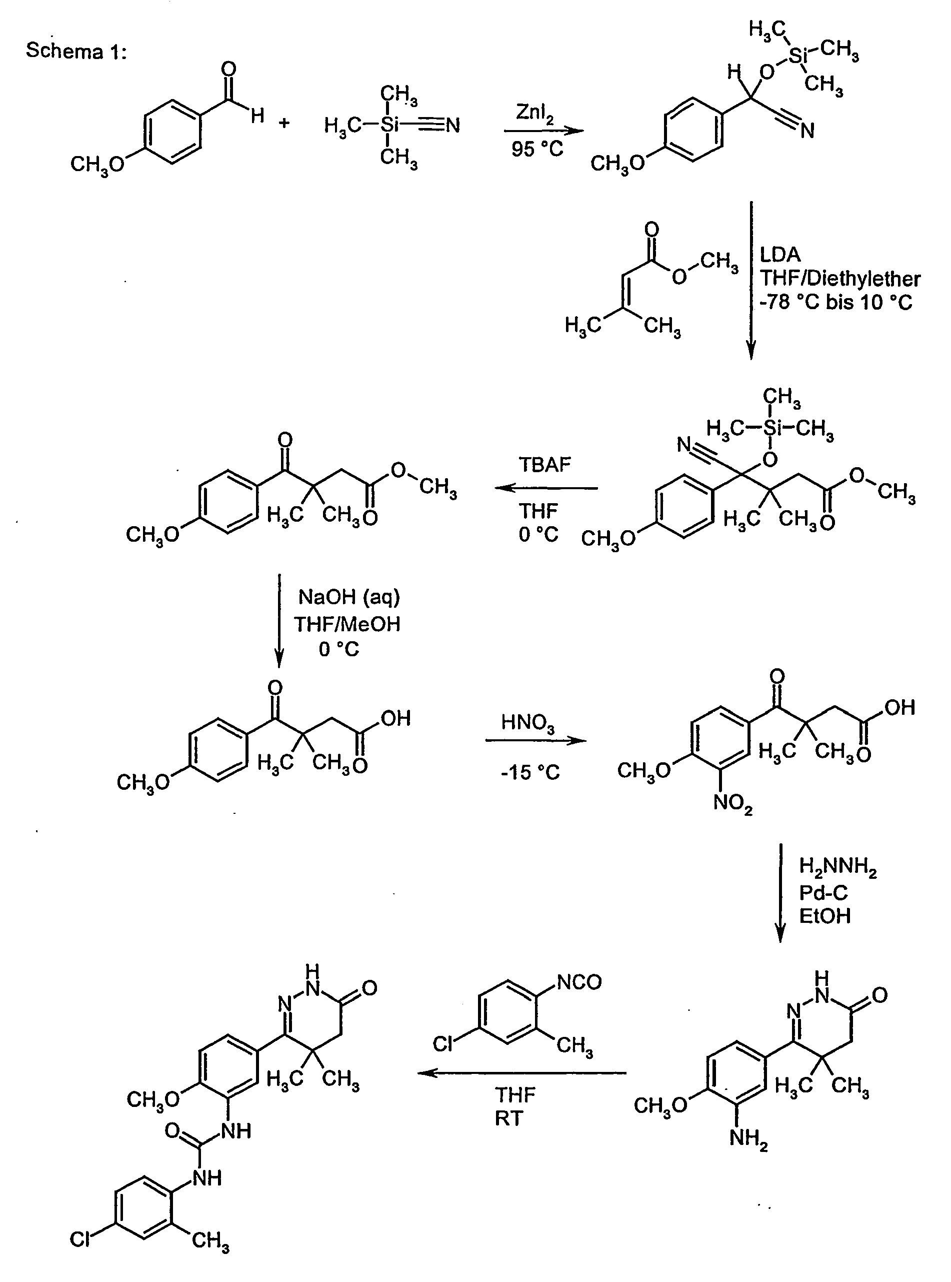

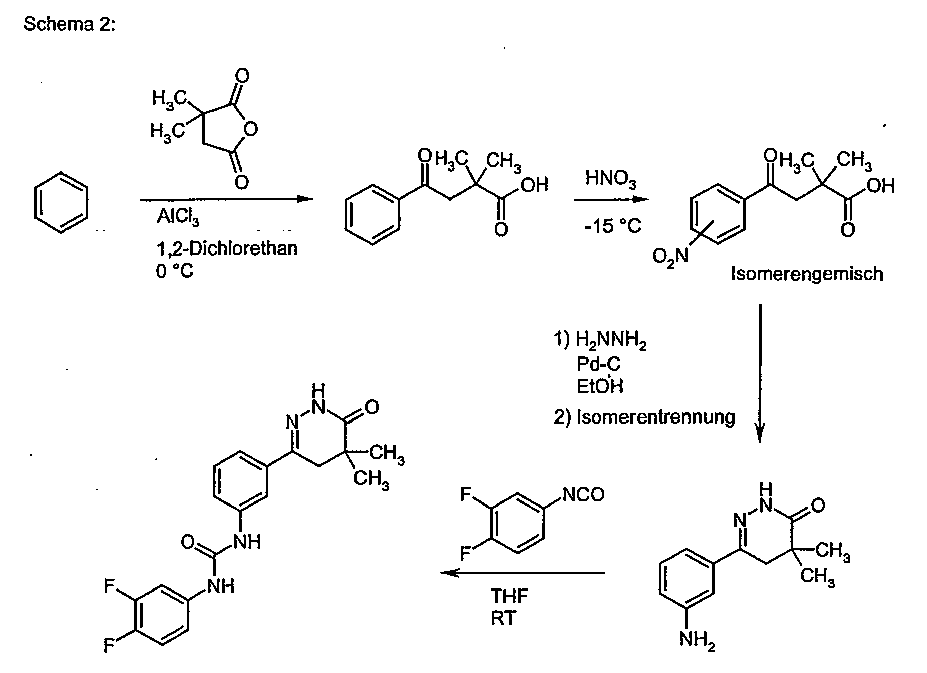

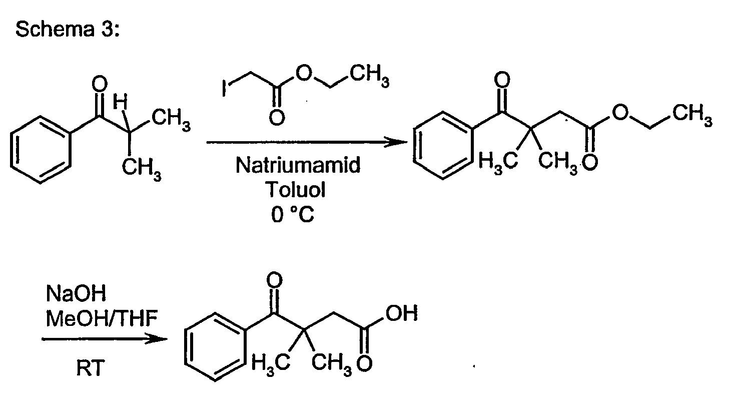

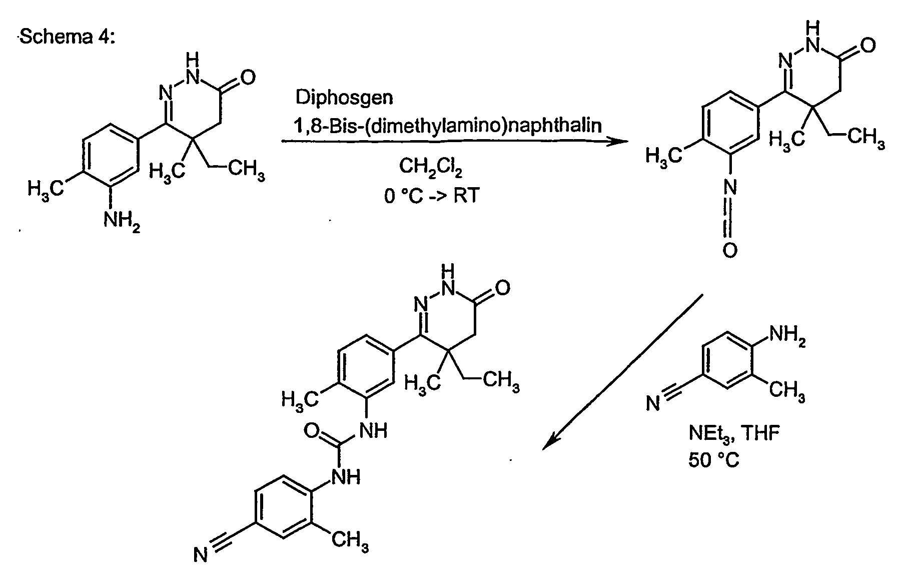

Bei Verfahren
[D] setzt man Verbindungen der allgemeinen Formel (IIb), 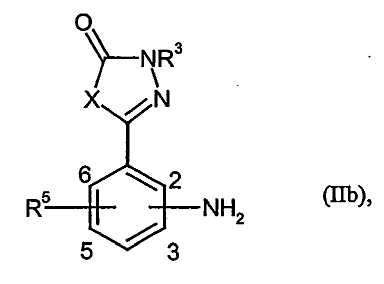
in welcher
NH₂ über eine der Positionen 2, 3, 5 oder 6 an den Aromaten gebunden ist, und
X, R³ und R⁵ die oben angegebene Bedeutung haben,
mit Verbindungen der Formel (IIIb)

DCN-R² (IIIb),

in welcher R² und D die oben angegebene Bedeutung haben, umgesetzt werden.

Die Umsetzung erfolgt in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt sind Tetrahydrofuran oder Methylenchlorid.

Basen sind beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt sind Diisopropylethylamin und Triethylamin.

Die Verbindungen der Formel (IIIb) sind bekannt oder lassen sich nach bekannten Verfahren aus den entsprechenden Edukten synthetisieren.

Die Verbindungen der Formel (IIab), welche Verbindungen der Formel (IIb) darstellen, worin X für 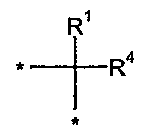 steht, können hergestellt werden, indem Verbindungen der Formel (IVb) 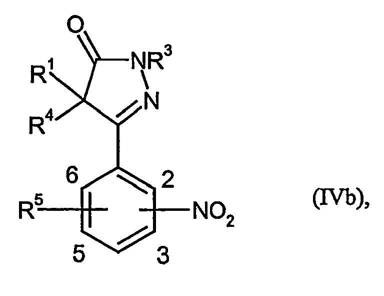 in welcher
NO₂ über eine der Positionen 2, 3, 5 oder 6 an den Aromaten gebunden ist, und
R¹, R³, R⁴ und R⁵ die oben angegebene Bedeutung haben,
reduziert werden, z.B. mit Zinn(II)-chlorid oder Wasserstoff mit Palladium auf Kohle.

Die Umsetzung erfolgt in inerten Lösungsmitteln, bevorzugt in einem Temperaturbereich von Raumtemperatur bis zum Rückfluss der Lösungsmittel bei Normaldruck bis 3 bar.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin; bevorzugt sind Ethanol, iso-Propanol oder im Falle von Zinndichlorid in Dimethylformamid.

Die Verbindungen der Formel (IVb) können hergestellt werden, indem Verbindungen der Formel (Vb) 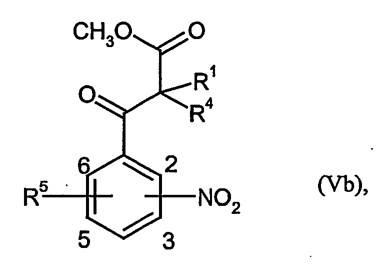 in welcher
NO₂ über eine der Positionen 2, 3, 5 oder 6 an den Aromaten gebunden ist, und
R¹, R⁴ und R⁵ die oben angegebene Bedeutung haben,
mit Hydrazin oder einer Verbindung der allgemeinen Formel (VIb), 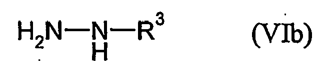 in welcher
R³ die oben angegebene Bedeutung aufweist, umgesetzt werden.

Die Umsetzung erfolgt in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, bevorzugt sind Ethanol oder iso-Propanol.

Die Verbindungen der Formel (VIb) sind bekannt oder lassen sich nach bekannten Verfahren aus den entsprechenden Edukten synthetisieren.

Die Verbindungen der Formel (Vb) können hergestellt werden, indem Verbindungen der Formel (VIIb) 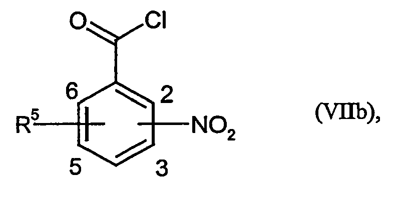 in welcher
NO₂ über eine der Positionen 2, 3, 5 oder 6 an den Aromaten gebunden ist, und
R⁵ die oben angegebene Bedeutung hat,
mit Verbindungen der Formel (VIIIb) 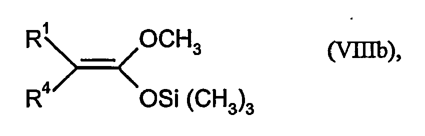 worin R¹ und R⁴ die oben angegebene Bedeutung haben,
in Gegenwart von Bortrifluoretherat umgesetzt werden.

Die Umsetzung erfolgt in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylacetamid, Acetonitril oder Pyridin, bevorzugt ist Diethylether.

Die Verbindungen der Formel (VIIb) sind bekannt oder können analog bekannten Verfahren hergestellt werden.

Die Verbindungen der Formel (VIIIb) sind bekannt oder können analog C. Ainsworth, F. Chen, Y.-N. Kuo, J. Organomet. Chem. 1972, 46, 59-71 hergestellt werden.

Die Verbindungen der Formel (IIbb), welche für Verbindungen der Formel (IIb) stehen, worin X für NR⁶ steht, können hergestellt werden, indem Verbindungen der Formel (IXb) 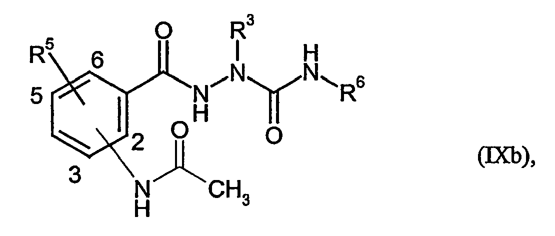 in welcher
NHC(O)CH₃ über eine der Positionen 2, 3, 5 oder 6 an den Aromaten gebunden ist, und
R³, R⁵ und R⁶ die oben angegebene Bedeutung haben,
in Wasser in Gegenwart einer Base, bevorzugt bei 60°C bis zum Rückfluss des Wassers bei Normaldruck umgesetzt werden.

Basen sind beispielsweise Alkalihydroxide wie Natrium-, Lithium- oder Kaliumhydroxid, Alkalicarbonate wie Cäsiumcarbonat, Natrium- oder Kaliumcarbonat, bevorzugt ist Natriumhydroxid.

Die Verbindungen der Formel (IXb) können hergestellt werden, indem Verbindungen der Formel (Xb) 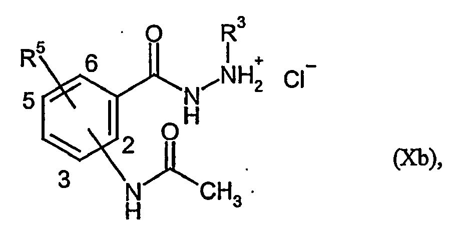 in welcher
NHC(O)CH₃ über eine der Positionen 2, 3, 5 oder 6 an den Aromaten gebunden ist, und
R³ und R⁵ die oben angegebene Bedeutung haben,
mit Verbindungen der Formel (XIb)

OCN-R⁶ (XIb),

in welcher R⁶ die oben angegebene Bedeutung hat,
nach dem für die Herstellung der Verbindungen der Formel (Ib) beschriebenen Verfahren umsetzt.

Die Verbindungen der Formel (XIb) sind bekannt oder können analog bekannten Verfahren hergestellt werden.

Die Verbindungen der Formel (Xb) können hergestellt werden, indem Verbindungen der Formel (XIIb) 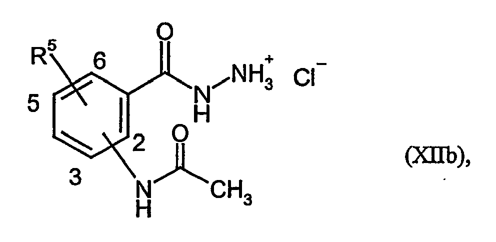 in welcher
NHC(O)CH₃ über eine der Positionen 2, 3, 5 oder 6 an den Aromaten gebunden ist, und
R⁵ die oben angegebene Bedeutung hat,
mit Verbindungen der Formel (XIIIb)

OHC-R³ (XIIIb)

in welcher R³ die oben angegebene Bedeutung hat,
in einer reduktiven Aminierung nach dem Fachmann für reduktive Aminierungen bekannten Verfahren umgesetzt werden.

Die Verbindungen der Formeln (XIIb) und (XIIIb) sind bekannt oder können analog bekannten Verfahren hergestellt werden.

Die Herstellung der erfindungsgemäßen Verbindungen kann durch die folgenden Syntheseschemata 5-12 verdeutlicht werden.

### Syntheseschemata:

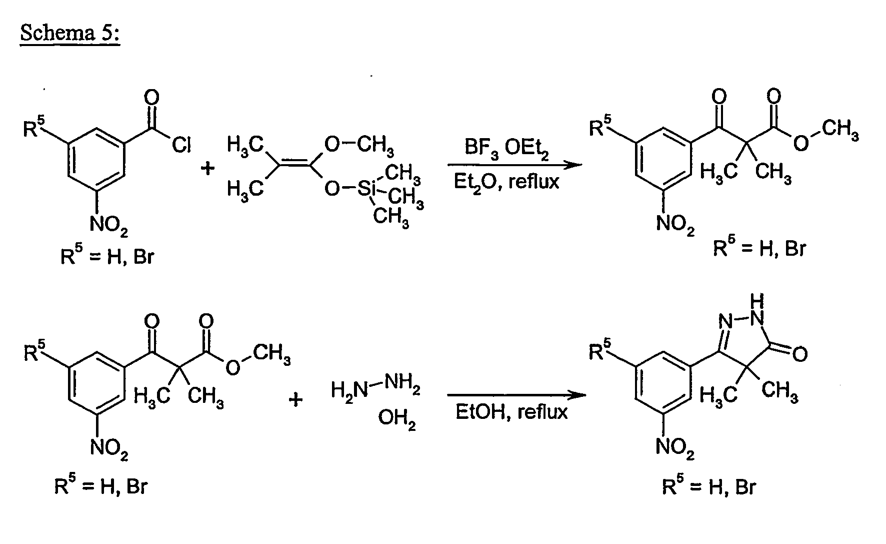

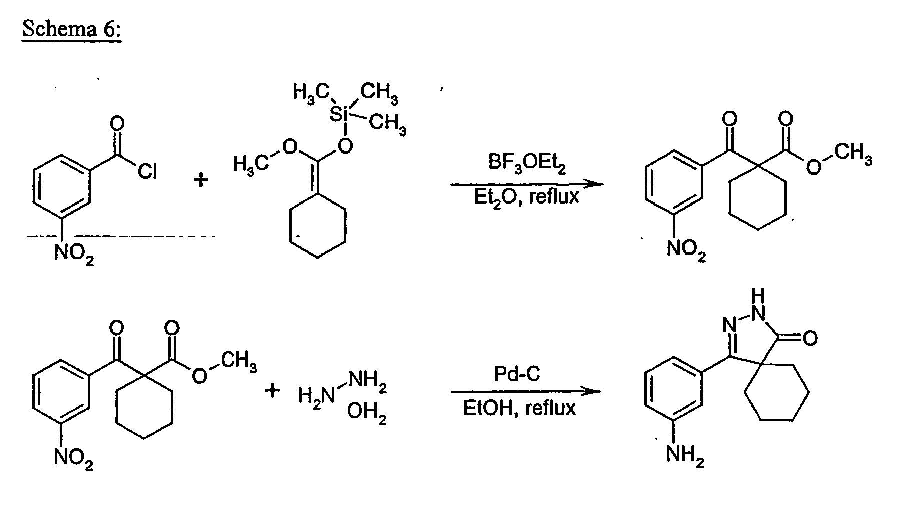

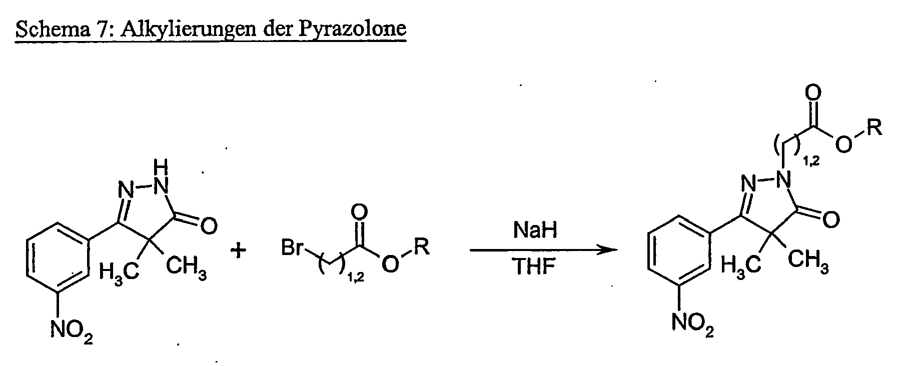

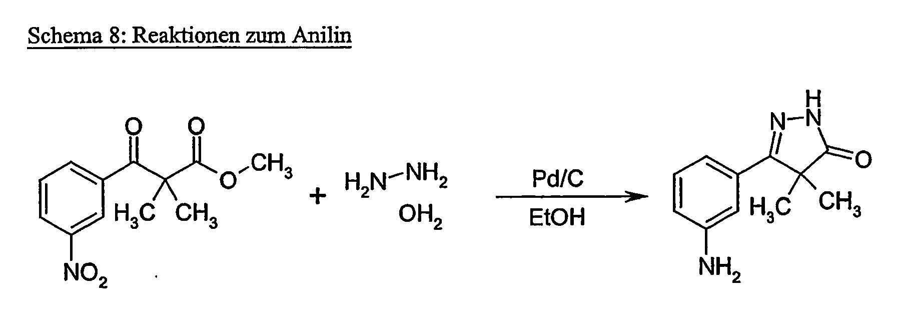

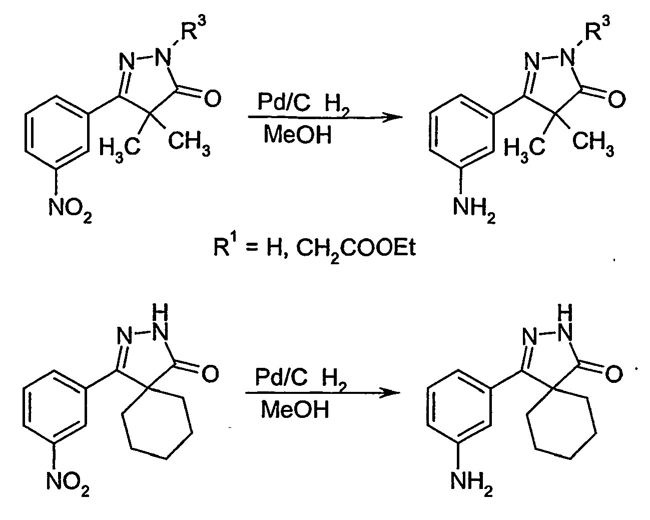

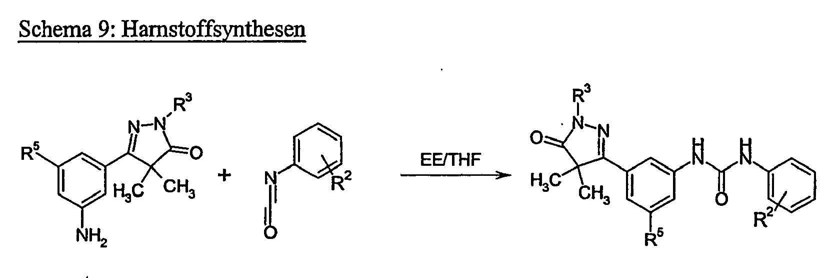

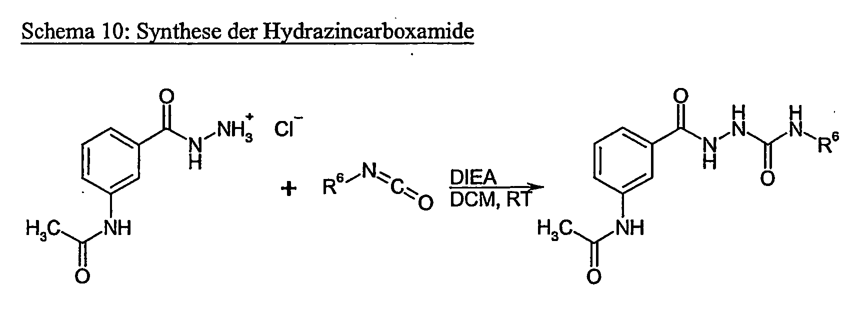

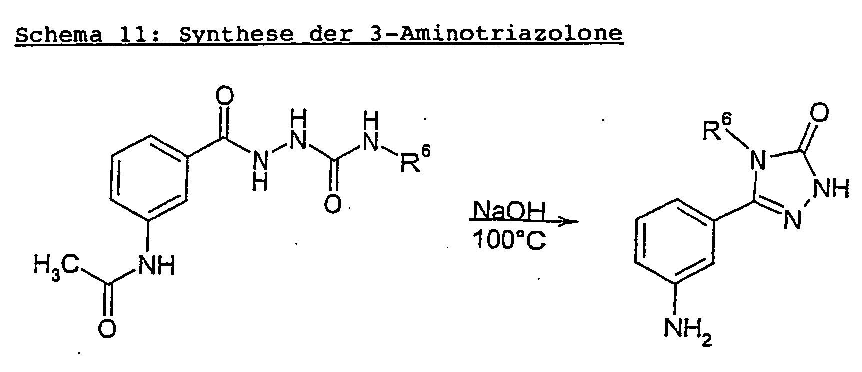

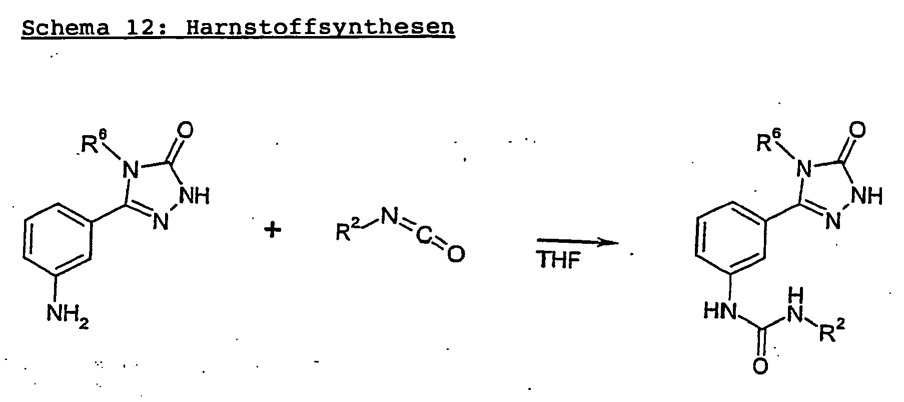

Weitere Verbindungen, die über den hier offenbarten Mechanismus wirken, sind in den internationalen Patentanmeldungen WO 03/097595 und WO 03/089421 beschrieben.

Die Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Sie zeichnen sich als UL86-Inhibitoren aus.

Die erfindungsgemäßen Verbindungen können aufgrund ihrer pharmakologischen Eigenschaften allein oder in Kombination mit anderen Wirkstoffen zur Behandlung und/oder Prävention von Herpesinfektionen, insbesondere von Infektionen mit dem humanen Cytomegalievirus (HCMV) eingesetzt werden.

Weiter beschrieben werden Arzneimittel, die mindestens eine erfindungsgemd8e Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen / -lösungen, Sprays; lingual, sublingual oder buccal zu applizierende Tabletten oder Kapseln, Suppositorien, Ohren- und Augen-präparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 5 mg/kg Körpergewicht zur Erzielung wirksamer Ergebnisse zu verabreichen. Bei oraler Applikation beträgt die Menge etwa 0.01 bis 25 mg/kg, vorzugsweise etwa 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

| | |
|---|---|
| Boc | Butoxycarbonyl |
| DC | Dünnschichtchromatographie |
| DCI | direkte chemische Ionisation (bei MS) |
| DCM | Dichlormethan |
| DIEA | *N,N*-Diisopropylethylamin |
| DMSO | Dimethylsulfoxid |
| DMF | Dimethylformamid |
| d. Th. | der Theorie |
| EE | Ethylacetat (Essigsäureethylester) |
| ESI | Elektrospray-Ionisation (bei MS) |
| Fp. | Schmelzpunkt |
| h | Stunde |
| HPLC | Hochdruck-, Hochleistungsflüssigchromatographie |
| LC-MS | Flüssigchromatographie-gekoppelte Massenspektroskopie |
| MS | Massenspektroskopie |
| moi | Multiplicity of infection |
| NMR | Kernresonanzspektroskopie |
| RP-HPLC | Reverse Phase HPLC |
| RT | Raumtemperatur |
| R_{f} | Retentionsindex (bei DC) |
| Rₜ | Retentionszeit (bei HPLC) |
| SI | Selektivitätsindex |
| THF | Tetrahydrofuran |

### HPLC- und LCMS-Methoden:

**Methode 1 (LCMS):** Instrument: Micromass Quattro LCZ, HP1100; Säule: Symmetry C18, 50 mm x 2.1 mm, 3.5 µm; Eluent A: Acetonitril + 0.1% Ameisensäure, Eluent B: Wasser + 0.1% Ameisensäure; Gradient: 0.0min 10%A → 4.0min 90%A → 6.0min 90%A; Ofen: 40°C; Fluss: 0.5ml/min; UV-Detektion: 208-400 nm.

**Methode 2 (HPLC):** Instrument: Finnigan MAT 900S, TSP: P4000,AS3000, UV3000HR; Säule: Symmetry C 18, 150 mm x 2.1 mm, 5.0 µm; Eluent C: Wasser, Eluent B: Wasser + 0.3g 35%ige Salzsäure, Eluent A: Acetonitril; Gradient: 0.0min 2%A → 2.5min 95%A → 5min 95%A; Ofen: 70 °C; Fluss: 1.2ml/mm; UV-Detektion: 210 nm.

**Methode 3:** Säule: Kromasil C18 60*2, L-R Temperatur: 30°C, Fluss = 0.75 mlmin⁻¹, Eluent: A = 0.005 M HClO₄, B = Acetonitril, Gradient: → 0.5 min 98 %A → 4.5 min 10 %A → 6.5 min 10 %A

### Ausgangsverbindungen

### Allgemeine Arbeitsvorschrift [A]:

### Synthese von TMS-Cyanhydrinen

Unter einer Argonatmosphäre werden in einem ausgeheizten 100 ml Dreihalskolben 55 mmol Trimethylsilylcyanid mit einer Spatelspitze wasserfreiem Zinkiodid versetzt. Bei RT werden 50 mmol der flüssigen Aldehyde langsam (exotherme Reaktion) zugetropft (feste Aldehyde werden bei 60°C als Feststoff portionsweise zugegeben). Die erhaltene braune Reaktionsmischung wird für 7-8 Stunden auf 95°C erwärmt. Danach wird das Produkt im Hochvakuum mit Hilfe eines Kugelrohrofens destilliert. Die dabei erhaltenen farblosen oder leicht gelben Flüssigkeiten werden ohne weitere Reinigung für die nächsten Umsetzungen verwendet.

Nach dieser Vorschrift wird folgende Verbindung hergestellt :

### Beispiel 1A

Phenyl[(trimethylsilyl)oxy]acetonitril

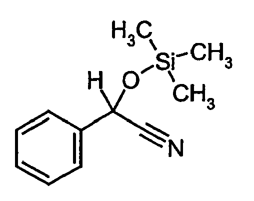

Ausgehend von 5.63 g (55 mmol) Trimethylsilylcyanid werden mit 5.31 g (50 mmol) Benzaldehyd 8.80 g (86% d. Th.) Produkt erhalten.
HPLC (Methode 3): Rₜ= 3.38 min
MS (DCI): m/z = 223 (M+NH₄)⁺

### Allgemeine Arbeitsvorschrift [B]:

### Umsetzung von TMS-Cyanhydrinen mit 3-Methyl-2-butensäuremethylester

1 eq. des entsprechenden TMS-Cyanhydrins wird in einem ausgeheizten 250 ml Dreihalskolben unter Argon in absolutem Diethylether gelöst und die erhaltene Lösung auf -78°C abgekühlt. Dazu werden 1.05 eq. 2 M LDA-Lösung in THF/Heptan/Ethylbenzol innerhalb von 30 min zugetropft. Man lässt noch 30 min bei dieser Temperatur rühren bevor 1 eq. 3-Methyl-2-butensäuremethylester, gelöst in wenig absolutem Diethylether, zugetropft wird. Innerhalb von 5 Stunden lässt man auf 0°C bis 10°C erwärmen. Daraufhin wird gesättigte Ammoniumchloridlösung zugegeben und 10 min gerührt. Die Phasen werden getrennt und die etherische Phase noch 2x mit gesättigter Ammoniumchloridlösung gewaschen. Nach Trocknung über Magnesiumsulfat und Filtration wird das Lösungsmittel am Rotationsverdampfer entfernt und man erhält das Produkt, das ohne weitere Reinigung für den nächsten Syntheseschritt eingesetzt wird.

Nach dieser Vorschrift wird folgende Verbindung hergestellt :

### Beispiel 2A

### 4-Cyano-3,3-dimethyl-4-phenyl-4-[(trimethylsilyl)oxy]butansäuremethylester

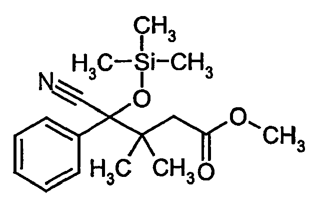

Ausgehend von 8.80 g (43 mmol) Phenyl[(trimethylsilyl)oxy]acetonitril werden, nach Deprotonierung mit 22.5 ml 2 M LDA-Lösung, mit 5.04 g (43 mmol) 3-Methyl-2-butensäuremethylester 13.69 g (67% d. Th.) der Titelverbindung als Rohprodukt erhalten.
HPLC (Methode 3): Rₜ= 5.53 min
MS (DCI): m/z = 337 (M+NH₄)⁺

### Allgemeine Arbeitsvorschrift [C]:

### Desilylierung mit Hilfe von TBAF

1 eq. der Butansäuremethylesterderivate wird unter einer Argonatmosphäre in absolutem THF (0.25 M) gelöst und auf 0°C abgekühlt. Bei dieser Temperatur werden 1.1 eq. einer 1 M TBAF-Lösung in THF langsam zugetropft. Man lässt noch 3 Stunden rühren, gibt dann Wasser zu und extrahiert 3x mit Dichlormethan. Nach Trocknung über Magnesiumsulfat, Filtration und Entfernung des Lösungsmittels wird säulenchromatographisch (Kieselgel: Laufmittel Cyclohexan/Ethylacetat = 85:15) oder mittels Kugelrohrdestillation gereinigt.

Nach dieser Vorschrift wird folgende Verbindung hergestellt:

### Beispiel 3A

### 3,3-Dimethyl-4-oxo-4-phenylbutansäuremethylester

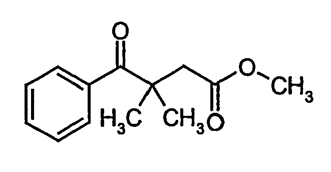

Ausgehend von 13.44 g (42 mmol) 4-Cyano-3,3-dimethyl-4-phenyl-4-[(trimethylsilyl)oxy]-butansäuremethylester werden mit 46.3 ml (46.3 mmol) einer 1 M TBAF-Lösung 6.54 g (62 % d. Th.) der Titelverbindung als Rohprodukt erhalten.
HPLC (Methode 3): Rₜ = 4.25 min
MS (DCI): m/z = 238 (M+NH₄)⁺

### Alternative Synthesemethode:

48.4 ml (24.20 mmol; 0.5 M Lösung in Toluol) Kaliumhexamethyldisilazid werden in 30 ml Tetrahydrofuran gelöst und bei -78°C mit 3.26 g (22 mmol) Isobutyrophenon in 10 ml Tetrahydrofuran versetzt. Nach 2 Stunden werden 4.04 g (26.40 mmol) Bromessigsäuremethylester dazugegeben. Nach weiteren 2 Stunden wird mit 50 ml 1N Salzsäure versetzt. Anschließend wird mit Essigsäureethylester extrahiert. Die vereinigten organischen Phasen werden mit Wasser und gesättigter Natriumchlorid-Lösung gewaschen, mit Magnesiumsulfat getrocknet und das Lösungsmittel entfernt. Nach präparativer Normal-Phasen-HPLC (Säule: Kieselgel, Fluss: 150ml/min, Eluent: iso-Hexan/Essigsäureethylester = 9:1) erhält man die Zielverbindung in einer Ausbeute von 26 %.
HPLC (Methode 3) Rₜ= 4.60 min
MS (DCI/NH₃): m/z = 238 (M+NH₄)⁺

### Allgemeine Arbeitsvorschrift [D]:

### Esterverseifung

Der zu verseifende Ester wird in einem THF/Methanol-Gemisch (1:1) gelöst und die Lösung auf 0°C abgekühlt. Bei dieser Temperatur werden 2 eq. 1 N Natronlauge langsam zugetropft. Nach beendeter Reaktion (Reaktionskontrolle mittels DC) werden jeweils gleiche Anteile einer 1N Natronlauge und Dichlormethan zugegeben. Die organische Phase wird zweimal mit 1 N Natronlauge extrahiert. Anschließend werden die vereinigten wässrigen Phasen mit konzentrierter Salzsäure angesäuert und das Produkt dreimal mit Dichlormethan extrahiert. Nach Trocknung über Natriumsulfat, Filtration und Verdampfen des Lösungsmittels wird das Produkt erhalten und ohne weitere Aufreinigung für den nächsten Syntheseschritt verwendet.

Nach dieser Vorschrift wird folgende Verbindung hergestellt:

### Beispiel 4A

### 5-Hydroxy-4,4-dimethyl-5-phenyldihydro-2(3H)-furanon

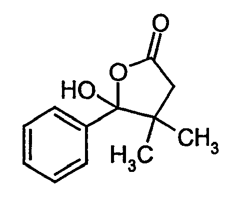

Ausgehend von 6.52 g (29.6 mmol) 3,3-Dimethyl-4-oxo-4-phenylbutansäuremethylester werden 5.20 g (83% d. Th.) Produkt erhalten.
HPLC (Methode 3): Rₜ= 3.88 min
MS (DCI): m/z = 224 (M+NH₄)⁺

### Beispiel 5A

### 5-Hydroxy-4,4-dimethyl-5-(3-nitrophenyl)dihydro-2(3H)-furanon und 5-Hydroxy-4,4-dimethyl-5-(4-nitrophenyl)dihydro-2(3H)-furanon

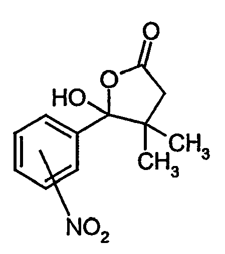

Rauchende Salpetersäure (12 ml) wird in einem Kolben unter Argon auf -15°C abgekühlt. Bei dieser Temperatur werden 5 g (24.5 mmol) 5-Hydroxy-4,4-dimethyl-5-phenyldihydro-2(3*H*)-furanon als Feststoff portionsweise zugegeben. Es wird noch eine halbe Stunde bei -15°C nachgerührt, dann auf Eis gegossen und dreimal mit Dichlormethan extrahiert. Die vereinigten Extrakte werden über Magnesiumsulfat getrocknet. Reinigung erfolgt mittels Säulenchromatographie (Dichlormethan-Methanol 97:3). Es werden 6.23 g eines Produktgemisches der Titelverbindungen als Rohprodukt erhalten.
HPLC (Methode 3): Rₜ= 4.06 min
MS (DCI): m/z = 269 (M+NH₄)⁺

### Beispiel 6A

### 6-(3-Aminophenyl)-5,5-dimethyl-4,5-dihydro-3(2H)-pyridazinon und 6-(4-Aminophenyl)-5,5-dimethyl-4,5-dihydro-3(2H)-pyridazinon

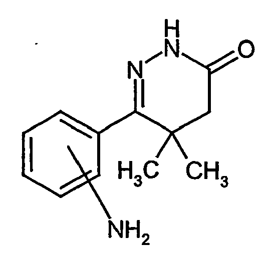

2.98 g (11.9 mmol) eines Gemisches aus 5-Hydroxy-4,4-dimethyl-5-(3-nitrophenyl)dihydro-2(3*H*)-furanon und 5-Hydroxy-4,4-dimethyl-5-(4-nitrophenyl)dihydro-2(3*H*)-furanon werden in 40 ml Ethanol bei RT gelöst und mit 8.91 g (178 mmol) Hydrazinmonohydrat versetzt. Daraufhin werden 300 mg Palladium/Kohle (10 Gew.-%) zugegeben und die Reaktionsmischung 20 Stunden zum Rückfluss erhitzt. Anschließend wird heiß über Celite filtriert, mit heißem Ethanol nachgewaschen und zur Trockene eingeengt. Aus Ethanol wird kristallisiert. Man erhält 1.09 g (34 % d. Th.) eines Produktgemisches mit 80 % meta- und 20 % para-Produkt. Erneute Kristallisation aus der Mutterlauge ergibt 1.03 g (30 % d. Th.) eines Produktgemisches mit 74 % para- und 26 % meta-Produkt. Die beiden Fraktionen werden vereinigt und an einer präparativen HPLC (Methode 12) in das para- und meta-Produkt getrennt.
HPLC (Methode 3): Rₜ= 2.53 min (para), bzw. 2.83 min (meta)
MS (EI): m/z = 217 (M)⁺

### Beispiel 7A

### 4-(2-Cyano-5-nitrophenyl)-3,3-dimethyl-4-oxobutansäure

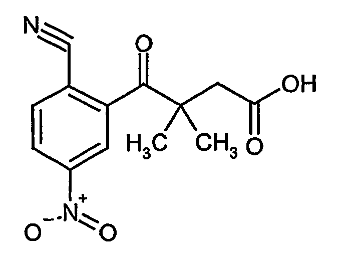

Die Herstellung von 4-(2-Cyano-5-nitrophenyl)-3,3-dimethyl-4-oxobutansäure erfolgt aus 4-(2-Fluor-5-nitrophenyl)-3,3-dimethyl-4-oxobutansäure in Anlehnung an die Literatur Heterocycles 1987, 26, 1227 und Synth. Commun. 1985, 15, 479.

### Beispiel 8A

### 6-(2-Hydroxy-5-nitrophenyl)-5,5-dimethyl-4,5-dihydro-3(2H)-pyridazinon

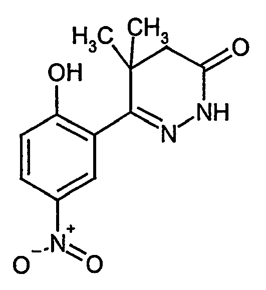

In 400 ml Ethanol werden 26.00 g (94.12 mmol) 4-(2-Cyano-5-nitrophenyl)-3,3-dimethyl-4-oxobutansäure gelöst und unter Rückfluss 47.12 g (941.19 mmol) Hydrazinhydrat zugetropft. Es wird 5 h in der Siedehitze gerührt und anschließend die Lösung bis auf 100 ml eingeengt. Der Rückstand wird mit Wasser versetzt und das Volumen auf 200 ml eingeengt. Anschließend werden die Kristalle abgesaugt und mit Wasser und Diethylether gewaschen. Nach Trocknen im Vakuum werden 20.03 g (81 % d. Th.) Produkt erhalten.
HPLC (Methode 3): Rₜ= 3.50 min
MS (DCI/NH₃): m/z =281 (M+NH₄)⁺.

### Beispiel 9A

### 6-(5-Amino-2-hydroxyphenyl)-5,5-dimethyl-4,5-dihydro-3(2H)-pyridazinon

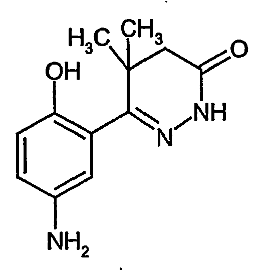

In 150 ml Ethanol werden 3.00 g (11.40 mmol) 6-(2-Hydroxy-5-nitrophenyl)-5,5-dimethyl-4,5-dihydro-3(2H)-pyridazinon gelöst und mit 0.30 g Palladium/Kohle (10 %) versetzt. In der Siedehitze werden 5.70 g (113.96 mmol) Hydrazinhydrat zugetropft. Nach 18 h Rühren unter Rückfluss wird das Lösungsmittel entfernt und der ölige Rückstand aus Diethylether kristallisiert. Es wird mit Wasser verrührt und die Kristalle abgesaugt. Nach Waschen mit Diethylether wird im Vakuum getrocknet. Es werden 1.84 g (69 % d. Th.) Produkt erhalten.
HPLC (Methode 3): Rₜ= 2.30 min
MS (ESI pos): m/z =234 (M+H)⁺.

### Beispiel 10A

### tert-Butyl 4-({[(3-chlor-4-fluorphenyl)amino]carbonyl}amino)phenylcarbamat

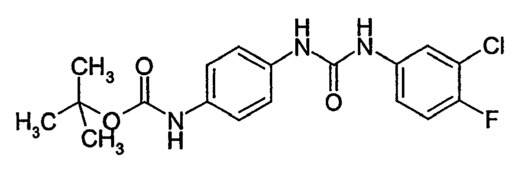

Zu einer mit Argon überschichteten, Suspension von 2.70 g (12.96 mmol) 4-(tert- Butoxycarbonyl-amino)anilin in 50ml Dichlormethan werden bei RT 2.34 g (13.61 mmol) 3-Chlor-4-fluorphenylisocyanat zugegeben. Es fällt sofort ein Niederschlag aus. Nachträglich werden noch 20 ml Dichlormethan zugegeben.

Nach einer Rührzeit von 30 min (DC1 Cyclohexan/Ethylacetat 1:1) wird der Niederschlag abgesaugt und mit Pentan gewaschen (DC2 Cyclohexan/Ethylacetat 1:1). Das Produkt wird bei 50°C am Rotationsverdampfer vorgetrocknet, bevor es im Hochvakuum getrocknet wird.
Man erhält 4.5g (90% d. Th.) Produkt.
R_{f}-Wert (Cyclohexan/Ethylacetat 1:1): 0.42
HPLC (Methode 1): Rₜ = 4.43 min

### Beispiel 11A

### N-(4-Aminophenyl)-N'-(3-chlor-4-fluorphenyl)harnstoff

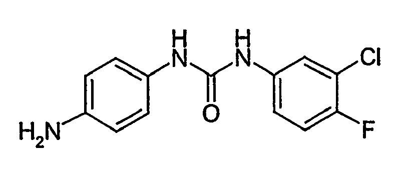

Eine Suspension von 4.53 g (11.93 mmol) Beispiel 10A in 50 ml 4N Chlorwasserstoff/Dioxan wird 2h unter Argon bei RT gerührt (DC1 Dichlormethan/Methanol 100:5). Der entstehende Niederschlag wird abgesaugt, mit Dioxan und Diethylether gewaschen (DC2 Dichlormethan/Methanol/Ammoniak 9:1:0.1). Das Produkt wird bei 50°C vorgetrocknet, bevor es im Hochvakuum getrocknet wird.
Man erhält 3.5g (quant.) Produkt.
R_{f}-Wert (Dichlormethan/Methanol/Ammoniak 9:1:0.1): 0.59
HPLC (Methode 1): Rₜ = 2.56min

### Beispiel 12A

### tert-Butyl 3-({[(3-chlor-4-fluorphenyl)amino]carbonyl}amino)phenylcarbamat

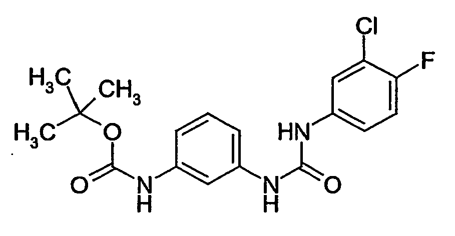

Zu einer Lösung von 3.00 g (14.41 mmol) 3-(tert-Butoxycarbonyl-amino)anilin in 50 ml Dichlormethan wird 2.59 g (15.13 mmol) 3-Chlor-4-fluorphenylisocyanat zugegeben. Es fällt nach wenigen Minuten ein Niederschlag aus. Es wird noch 2h bei RT gerührt (DC1 Cyclohexan/Ethylacetat 1:1).

Der Niederschlag wird abgesaugt, mit Dichlormethan und Diisopropylether gewaschen (DC2 Cyclohexan/Ethylacetat 1: 1) und im Hochvakuum getrocknet.
R_{f}-Wert (Cyclohexan/Ethylacetat 1:1): 0.63
HPLC (Methode 2): Rₜ = 2.95min

### Beispiel 13A

### N-(3-Aminophenyl)-N'-(3-chlor-4-fluorphenyl)harnstoff

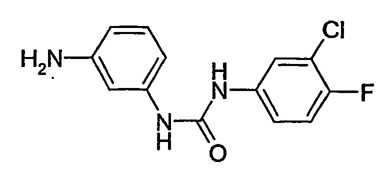

Eine Suspension von 5.00 g (13.16 mmol) Beispiel 12A in 100ml 4N Chlorwasserstoff/Dioxan wird 17h bei RT gerührt (DC1 Dichlormethan/Methanol 100:5). Der Feststoff wird abgesaugt, mit Dioxan und Diethylether gewaschen (DC2 Dichlormethan/Methanol 100:5) und im Hochvakuum 2 Tage getrocknet.
Man erhält 4.6g (quant.) Produkt.
R_{f}-Wert (Dichlormethan/Methanol 100:5): 0.14
HPLC (Methode 1): Rₜ = 3.01min

### Allgemeine Arbeitsvorschrift [E]:

Synthese von β-Ketoestern (analog Vorschrift von M. H. Stefaniak, F. Tinardon, J. D. Wallis, Synlett 1997, 677-678).

Unter einer Argonatmosphäre werden in einem ausgeheizten 500 ml Dreihalskolben 1 Äquivalent des entsprechend substituierten 3-Nitrobenzoesäurechlorids in absolutem Diethylether (0.25 M Lösung) gelöst und mit 1 Äquivalent 1-Methoxy-2-methyl-1-trimethylsiloxypropen (C. Ainsworth, F. Chen, Y.-N. Kuo, J. Organomet. Chem. 1972, 46, 59-71) versetzt. Nach Zugabe von einem Äquivalent (gegebenenfalls 3 Äquivalenten) Bortrifluorid-Diethylether-Komplex wird 24 Stunden zum Rückfluss erhitzt. Nach Erkalten der Reaktionsmischung wird jeweils einmal mit 1N Natronlauge, Wasser und gesättigter Kochsalzlösung gewaschen. Die organische Phase wird über Magnesiumsulfat getrocknet. Nach Filtration und Entfernung des Lösungsmittels erfolgt säulenchromatographische Reinigung des Rohproduktes (Kieselgel: Cyclohexan-Essigsäureethylester 9:1).

### Beispiel 14A

### 2,2-Dimethyl-3-(3-nitrophenyl)-3-oxopropansäuremethylester

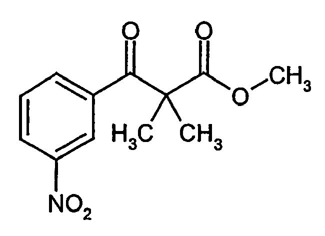

Ausgehend von 10 g (53.9 mmol) 3-Nitrobenzoesäurechlorid werden mit 9.40 g (53.9 mmol) 1-Methoxy-2-methyl-1-trimethylsiloxypropen und 7.65 g (53.9 mmol) Bortrifluorid-Diethylether-Komplex 4.93 g (25 % d. Th) Produkt erhalten.
HPLC (Methode 3): Rₜ= 4.49 min
MS (DCI): m/z = 269 (M+NH₄)⁺

### Allgemeine Arbeitsvorschrift [F]: Pyrazolonsynthesen

1 Äquivalent des β-Ketoesters wird zusammen mit 5 Äquivalenten Hydrazinhydrat in Ethanol (0.23 M Lösung) für 4 Stunden zum Rückfluss erhitzt. Dabei fällt das Reaktionsprodukt entweder aus der Reaktionsmischung aus oder wird nach Entfernung eines Teils des Lösungsmittels mit Wasser und Cyclohexan ausgefällt. Der Niederschlag wird abgesaugt, mit Diethylether gewaschen und dann im Vakuum getrocknet.

### Beispiel 15A

### 4,4-Dimethyl-5-(3-nitrophenyl)-2,4-dihydro-3H-pyrazol-3-on

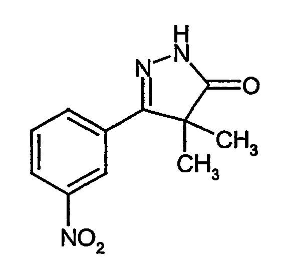

Ausgehend von 8.53 g (34 mmol) 2,2-Dimethyl-3-(3-nitrophehyl)-3-oxopropansäure-methylester werden mit 8.50 g (170 mmol) Hydrazinhydrat 6.63 g (83 % d. Th.) Produkt erhalten.
Fp.: 164.6°C
HPLC (Methode 3): Rₜ= 3.99 min
MS (DCI): m/z = 251 (M+NH₄)⁺

### Allgemeine Arbeitsvorschrift [G]: Katalytische Hydrierung der Nitrogruppe am Aromaten

20 mmol der zu hydrierenden Substanz werden in 100 ml entgastem Methanol gelöst und dann unter Argon mit 250 mg Palladium auf Aktivkohle versetzt. Unter einer Wasserstoffatmosphäre (Normaldruck) wird so lange hydriert bis DC-Kontrolle vollständigen Umsatz anzeigt. Danach wird über Kieselgur abgesaugt, das Filtrat eingeengt, der Rückstand im Vakuum getrocknet und ohne weitere Reinigung weiterverarbeitet.

### Beispiel 16A

### 4,4-Dimethyl-5-(3-aminophenyl)-2,4-dihydro-3H-pyrazol-3-on

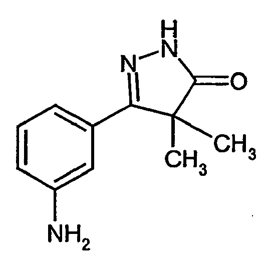

Ausgehend von 4.60 g (19.2 mmol) 4,4-Dimethyl-5-(3-nitrophenyl)-2,4-dihydro-3*H*-pyrazol-3-on werden 3.66 g (91 % d. Th.) Produkt erhalten.
HPLC (Methode 3): Rₜ= 3.04 min
MS (ESIpos): m/z = 204 (M+H)⁺

### Allgemeine Arbeitsvorschrift [H]: Synthese der Hydrazincarboxamide

1 Äquivalent von 2-[3-(Acetylamino)benzoyl]hydraziniumchlorid wird in Dichlormethan vorgelegt (0.15 M Lösung) und zusammen mit 2 Äquivalenten Diisopropylethylamin und 1 Äquivalent des entsprechenden Isocyanates 16 Stunden bei Raumtemperatur gerührt. Der entstandene Niederschlag wird abgesaugt, mit Diethylether gewaschen und im Vakuum getrocknet. Das Rohprodukt wird daraufhin direkt weiter umgesetzt.

### Beispiel 17A

### 2-[3-(Acetylamino)benzoyl]-N-isopropylhydrazincarboxamid

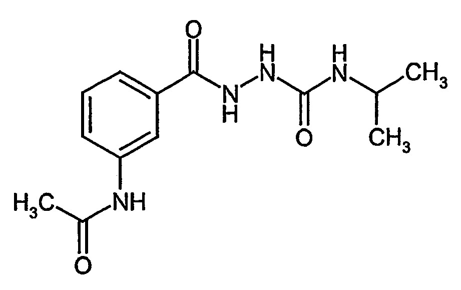

7.00 g (30.48 mmol) 2-[3-(Acetylamino)benzoyl]hydraziniumchlorid werden mit 7.88 g (60.96 mmol) Diisopropylethylamin und 2.59 g (30.48 mmol) Isopropylisocyanat umgesetzt. Das Rohprodukt wird daraufhin direkt weiter umgesetzt.
HPLC (Methode 3): Rₜ = 2.95 min

### Beispiel 18A

### 2-[3-(Acetylamino)benzoyl]-N-cyclohexylhydrazincarboxamid

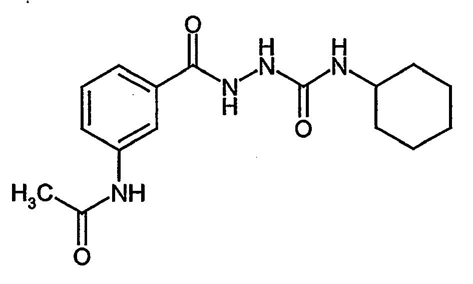

7.00 g (30.48 mmol) 2-[3-(Acetylamino)benzoyl]hydraziniumchlorid werden mit 7.88 g (60.96 mmol) Diisopropylethylamin und 3.82 g (30.48 mmol) Cyclohexylisocyanat umgesetzt. Das Rohprodukt wird daraufhin direkt weiter umgesetzt.
HPLC (Methode 3): Rₜ = 3.46 min

### Allgemeine Arbeitvorschrift [I]: Synthese der 3-Aminotriazolone

1 Äquivalent des entsprechenden Hydrazincarboxamids wird in 1 N Natronlauge gelöst (0.16 M Lösung) und mit 6.15 Äquivalenten Natriumhydroxid versetzt. Man lässt 48 Stunden bei 100°C rühren. Die Reaktionslösung wird mit Salzsäure auf pH 7 eingestellt, der entstandene Niederschlag wird abgesaugt, mit Wasser gewaschen und im Vakuum getrocknet.

### Beispiel 19A

### 5-(3-Aminophenyl)-4-isopropyl-2,4-dihydro-3H-1,2,4-triazol-3-on

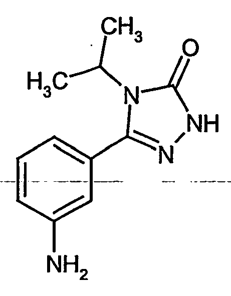

Ausgehend von 6.06 g (32.55 mmol) 2-[3-(Acetylamino)benzoyl]-N-isopropyl-hydrazincarboxamid (roh) und 8.00 g (200.02 mmol) Natriumhydroxid in 200 ml 1 N Natronlauge werden 2.81 g (40 % d. Th.) Produkt erhalten.
HPLC (Methode 3): Rₜ = 2.76 min

### Beispiel 20A

### 5-(3-Aminophenyl)-4-cyclohexyl-2,4-dihydro-3H-1,2,4-triazol-3-on

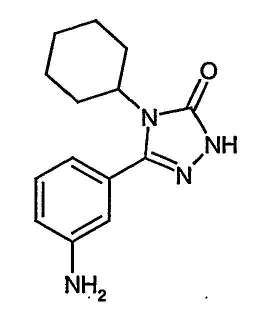

Ausgehend von 7.43 g (32.55 mmol) 2-[3-(Acetylamino)benzoyl]-N-cyclohexyl-hydrazincarboxamid (roh) und 8.00 g (200.02 mmol) Natriumhydroxid in 200 ml 1 N Natronlauge werden 5.59 g (66 % d. Th.) Produkt erhalten.
HPLC (Methode 3): Rₜ = 3.31 min

### Beschreibung der Abbildung

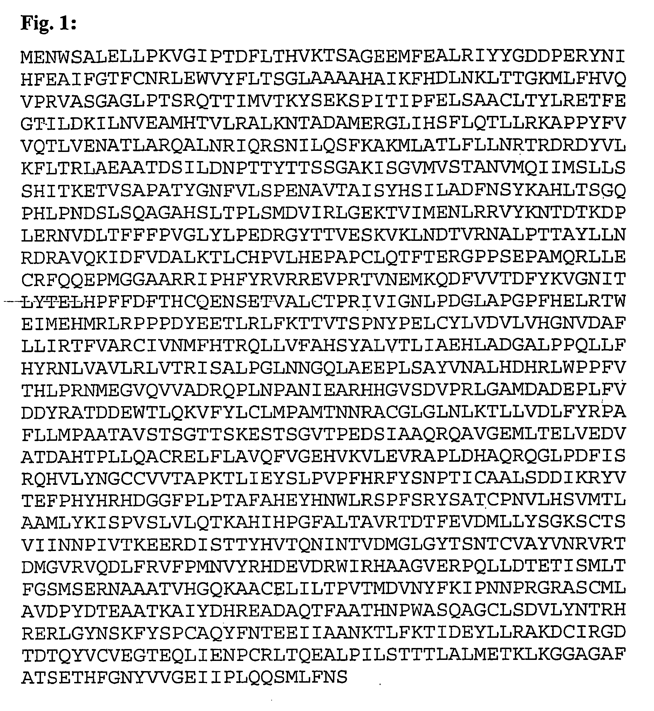

**Fig. 1:** Fig. 1 zeigt die Aminosäuresequenz des Wildtyp HCMV UL86 Proteins (Acc.-No. P16729, SEQ ID NO: 1)

### Ausführungsbeispiele

### Beispiel 1

### N-(2,4-Difluorphenyl)-N'-[3-(4,4-dimethyl-6-oxo-1,4,5,6-tetrahydro-3-pyridazinyl)phenyl]harnstoff

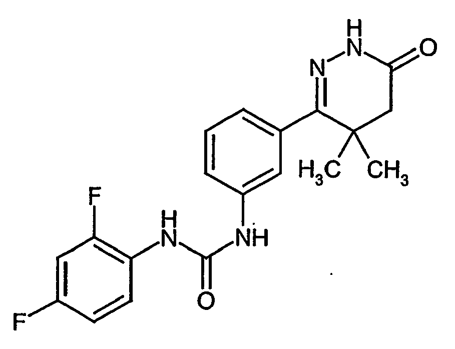

Bei Raumtemperatur werden 50 mg (0.23 mmol) 6-(3-Aminophenyl)-5,5-dimethyl-4,5-dihydro-3(2*H*)-pyridazinon mit 2 ml abs. THF versetzt und anschließend werden 71.4 mg (0.46 mmol) 2,4-Difluorphenylisocyanat zugegeben. Anfangs löst sich das 6-(3-Aminophenyl)-5,5-dimethyl-4,5-dihydro-3(2*H*)-pyridazinon nicht vollständig. Erst nach Zugabe des Isocyanates erhält man nach kurzer Zeit eine klare gelbe Lösung, aus der jedoch rasch ein weißer Niederschlag ausfällt. Man lässt über Nacht rühren und filtriert dann den Niederschlag ab. Mit Diethylether wird nachgewaschen und der weiße Feststoff im Vakuum getrocknet. Es werden 46.4 mg (54 % d. Th.) Produkt erhalten.
Fp.: 213°C
¹H-NMR (200 MHz, DMSO): δ = 1.16 (s, 6H), 2.35 (s, 2H), 6.97-7.11 (m, 2H), 7.25-7.39 (m, 3H), 7.65 (s, 1H), 7.99-8.17 (m, 1H), 8.50 (s, br 1H), 9.12 (s, br 1 H), 10.99 (s, 1H).
HPLC (Methode 3): Rₜ= 4.12 min
MS (ESIpos): m/z = 373 (M+H)⁺

### Beispiel 2

### N-(2,5-Difluorphenyl)-N'-[3-(4,4-dimethyl-6-oxo-1,4,5,6-tetrahydro-3-pyridazinyl)-4-hydroxyphenyl]harnstoff

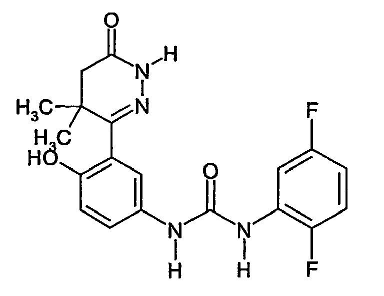

Die Synthese erfolgt analog zu Beispiel 1 aus den entsprechenden Edukten.
HPLC (Methode 3): Rₜ= 4.00 min
MS (ESIpos): m/z = 389 (M+H)⁺

### Beispiel 3

### N-[4-({[(3-Chlor-4-fluorphenyl)amino]carbonyl}amino)phenyl]acetamid

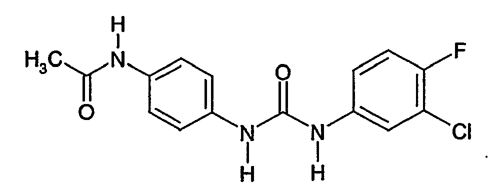

Die Verbindung ist bei der Firma Salor (Deisenhofen, Deutschland, Art.-Nr. S90,580-1) käuflich zu erwerben.

### Beispiel 4

### N-[4-({[(3-Chlor-4-fluorphenyl)amino]carbonyl}amino)phenyl]pentanamid

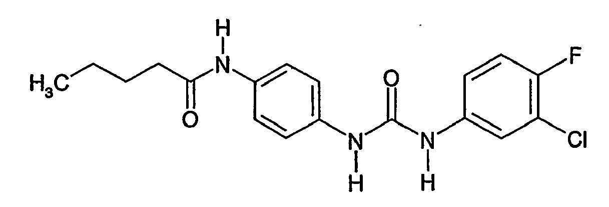

Zu einer Lösung von 100.0 mg (0.358 mmol) Beispiel 11A in 5 ml DMF werden 70.7 mg (0.894 mmol) Pyridin und 64.7 mg (0.536 mmol) Pentanoylchlorid zugegeben. Es wird 18h bei RT gerührt. Nach Zugabe von Wasser fällt ein weißer Niederschlag aus, der abgesaugt und mit Wasser und Pentan gewaschen wird. Das Produkt wird im Hochvakuum getrocknet. Man erhält 62 mg (80% d. Th.) Produkt.
R_{f} (Cyclohexan/Ethylacetat 1:3): 0.44
HPLC (Methode 2): Rₜ = 2.61 min
¹H-NMR (300MHz, d₆-DMSO): δ = 9.72 (s, 1H, NH), 8.79 (s, 1H, NH), 8.62 (s, 1H, NH), 7.83-7.74 (m, 1H, C₆H₃ClF), 7.55-7.32 (m, 4H, p-C₆H₄), 7.32-7.25 (m, 2H, C₆H₃ClF), 2.27 (t, 2H, CH₂), 1.58 (q, 2H, CH₂), 1.32 (sext, 2H, CH₂), 0.90 (t, 3H, CH₃).

### Beispiel 5

### N-[3-({[(3-Chlor-4-fluorphenyl)amino]carbonyl}amino)phenyl]-1-butansulfonamid

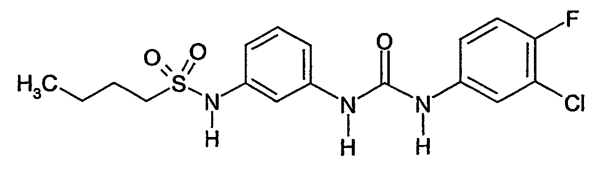

Zu einer Lösung von 200.0 mg (0.715 mmol) Beispiel 13A in einem Gemisch aus 2 ml DMF und 5 ml THF werden 169.7 mg (2.145 mmol) Pyridin und 168.0 mg (1.073 mmol) 1-Butansulfonylchlorid zugegeben. Es wird über Nacht bei RT gerührt. Nach Zugabe von Wasser fällt kein Niederschlag aus. Die Mischung wird im Vakuum eingeengt. Das Produkt wird über HPLC gereinigt (RP18-Säule; Laufmittel: Acetonitril-Wasser, Gradient 15:85->85:15). Man erhält 51 mg (18% d. Th.) Produkt.
R_{f} (Cyclohexan/Ethylacetat 1:2): 0.51
HPLC (Methode 1): Rₜ = 4.28 min
¹H-NMR (300MHz, d₆-DMSO): δ = 9.74 (s, 1H, NH), 8.85 (s, 1H, NH), 8.78 (s, 1H, NH), 7.83-7.76 (m, 1H, C₆H₃ClF), 7.38-7.27 (m, 3H, m-C₆H₄, C₆H₃ClF), 7.24-7.18 (m, 2H, m-C₆H₄), 6.87-6.78 (m, 1H, m-C₆H₄), 3.08 (t, 2H, CH₂); 1.65 (q, 2H, CH₂), 1.35 (sext, 2H, CH₂), 0.83 (t, 3H, CH₃).

### Allgemeine Arbeitsvorschrift [J]: Harnstoffe

1 Äquivalent des Anilins wird in THF vorgelegt (0.14 M Lösung) und mit 1 Äquivalent des entsprechenden Isocyanates versetzt. Die Lösung wird 1 Stunde bei Raumtemperatur geschüttelt.

Das Lösungsmittel wird im Vakuum entfernt und das Produkt mittels präparativer HPLC gereinigt (CromSil C 18, 250x30, Fluss: 50 ml/min, Laufzeit: 38 min, Detektion bei 210 nm, Gradient: 10% Acetonitril (3 min)->90 % Acetonitril (31 min)->90 % Acetonitril (34 min)->10% Acetonitril (34.01 min)).

Die Beispiele 6 und 7 können nach der allgemeinen Synthesemethode [J] hergestellt werden.

| **Beispiel** | **Struktur** | **Molekulargewicht** | **MS (ESI+) m/z** | **HPLC Rₜ[min]** | **HPLC-Methode** |
|---|---|---|---|---|---|
| 6 | 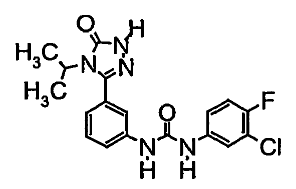 | 389.82 | 390 | 4.25 | 3 |
| 7 | 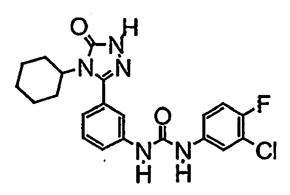 | 429.88 | 430 | 4.61 | 3 |

### Beispiel 8

### N-(4-Chlor-2-methylphenyl)-N-[3-(4,4-dimethyl-5-oxo-4,5-dihydro-1H-pyrazol-3-yl)phenyl]harnstoff

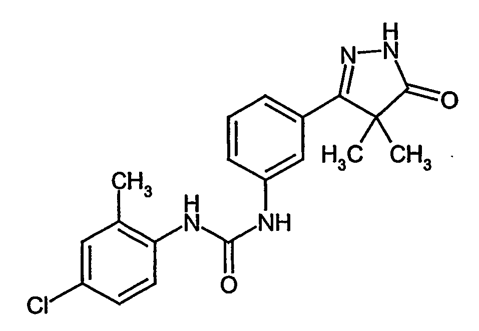

46.2 mg (0.28 mmol) 4-Chlor-2-methylphenylisocyanat werden mit einer Lösung von 40 mg 5-(3-Aminophenyl)-4,4-dimethyl-2,4-dihydro-3*H*-pyrazol-3-on in 1 ml Essigsäureethylester und 0.2 ml Tetrahydrofuran versetzt und über Nacht bei Raumtemperatur gerührt. Dabei beobachtet man die Bildung eines weißen Niederschlags.

Aufarbeitung: Die Reaktionsmischung wird eingeengt und der erhaltene Rückstand nach Aufnahme in DMSO mittels RP-HPLC gereinigt. So erhält man 42 mg (58 % d.Th.) Produkt.
Fp.: 226.8°C
HPLC (Methode 3): Rₜ= 4.33 min
MS (ESIpos): m/z = 371 (M+H)⁺
¹H-NMR (200 MHz, DMSO): δ = 1.37 (s, 6H), 2.25 (s, 3H), 7.21 (dd, 1H), 7.28 (d, 1H), 7.35-7.46 (m, 3H), 7.88 (d, 1H), 8.02 (s br, 1H), 8.11 (s br, 1H), 9.23 (s br, 1H), 11.54 (s br, 1H)

### B. Bewertung der physiologischen Wirksamkeit

Die *in vitro*-Wirkung der erfindungsgemäßen Verbindungen kann im folgenden Assay gezeigt werden:

### Virusanzucht:

Humanes Cytomegalievirus (HCMV), Stamm DavisSmith (ATCC VR807) oder AD169 (ATCC VR538), wird *in vitro* auf humanen embryonalen Vorhautfibroblasten (NHDF-Zellen) angezüchtet. Nach Infektion der NHDF-Zellen mit einer Multiplizität der Infektion (M.O.I) von 0,01 werden die virusinfizierten Zellen 5-10 Tage später geerntet und in Gegenwart von Minimal Essential Medium (MEM), 10 % foetalem Kälberserum (FKS) mit 10 % DMSO bei -80°C aufbewahrt. Zur Herstellung eines zellfreien Stocks wird nur der Zellkulturüberstand abgenommen und direkt bei -80°C eingefroren. Nach serieller Verdünnung der virusinfizierten Zellen oder des Zellkulturüberstandes der virusinfizierten Zellen (zellfreies Virus) in Zehnerschritten erfolgt die Titerbestimmung auf 24-Well-Platten konfluenter NHDF-Zellen nach Vitalfärbung mit Neutralrot.

### Anti-HCMV- (Auti-Humanes Cytomegalo-Virus) Zytopathogenitätstests

Die Testverbindungen werden als 50 millimolare (mM) Lösungen in Dimethysulfoxid (DMSO) eingesetzt. Ganciclovir^{®}, Foscarnet^{®} und Cidofovir^{®} dienen als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0,001 - 0,002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0,0005 µM. Die Platten werden 6 Tage bei 37°C / 5 % CO₂ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet. Für die erfindungsgemäßen Substanzen ergaben sich die in Tabelle 1 aufgeführten IC₅₀-Werte:

**Tabelle 1: Antivirale Wirksamkeit in vitro**

| **Beispiel** | **IC50[µM]** | **SI** |
|---|---|---|
| 1 | 1 | 125 |
| 2 | 0,4 | 325 |
| 3 | 0,5 | 40 |
| 4 | 0,08 | 750 |
| 5 | 0,14 | 70 |
| 6 | 0,4 | 150 |
| 7 | 1,5 | 30 |
| 8 | 0,6 | 30 |

### Selektion und Analyse von resistenten Mutanten

Dazu werden NHDF-Zellen in Gewebekulturgefäßen ausgesät. 5 x 10³ Zellen pro well werden in 96-well-Platten ausgesät und mit zellfreiem HCMV AD169 infiziert mit einer moi von 0,03. Die Infektionen werden unter Substanzdruck kultiviert, der dem 10-fachen IC₅₀ Wert der Substanz entspricht. Bevorzugterweise werden 30-100 wie beschrieben beimpfter 96-well-Platten angesetzt. Wells, die einen cytopathischen Effekt (CPE) vergleichbar einer Virusinfektion ohne Substanzdruck aufweisen, werden weiter analysiert, d.h. der die Viren enthaltende Wellinhalt (Zellen und Zellkulturüberstand) wird auf frischen Zellkulturen passagiert und weiter unter Substanzdruck kultiviert. Schließlich werden, wie oben beschrieben, Virenstocks hergestellt und eingefroren. Nach serieller Verdünnung der virusinfizierten Zellen oder des Zellkulturüberstandes der virusinfizierten Zellen in Zehnerschritten erfolgt die Titerbestimmung auf 24-Well-Platten konfluenter NHDF-Zellen nach Vitalfärbung mit Neutralrot. Schließlich erfolgt die Bestimmung der Suszeptibilität gegenüber diversen Substanzen (IC₅₀-Wert Bestimmung) wie oben beschrieben.

### Bestimmung der UL86-Sequenz

Die wie oben beschrieben selektierten HCMVAD169 Mutanten werden *in vitro* auf humanen embryonalen Vorhautfibroblasten (NHDF-Zellen) unter Substanzdruck (10x IC₅₀ HCMVAD169) angezüchtet. Nach Infektion der NHDF-Zellen mit einer Multiplizität der Infektion (M.O.I) von 0,01 werden die virusinfizierten Zellen 5-10 Tage später geerntet und es erfolgt eine Isolierung der Gesamt-DNA aus diesen Zellen mit Hilfe von etablierten Standardmethoden (z.B. Phenolextraktion und Ethanolpräzipitation). Qiagen Sequencing Services (Qiagen, Hilden) bestimmte nach Amplifikation des viralen UL86 Gens mittels PCR, die DNA-Sequenz, welche dann in Proteinsequenz umgeschrieben wurde. Im Vergleich zum Ausgangsstamm HCMV AD169 konnten bei den resistenten Viren die in Tabelle 2 dargestellten Abweichungen in der Proteinsequenz des Major Capsid Proteins (UL86) festgestellt werden. Die IC₅₀-Werte [µM] diverser Substanzen bei diesen Mutanten im Vergleich zum Ausgangsstamm sind ebenfalls angegeben. (n.d. = nicht gemessen). Die auf UL86 einwirkenden Substanzen zeigen bei den UL86-Mutanten eine stark verringerte Wirksamkeit, wohingegen der DNA-Replikationsinhibitor Ganciclovir als Kontrolle unverändert wirksam ist. Je nach Art der Substanz führen bestimmte Mutationen zu einer verschieden stark ausgeprägten Resistenz. Die Fälle, bei denen ein >10x erhöhter IC₅₀-Wert festgestellt werden konnte sind fett und unterstrichen dargestellt.

Die Eignung der erfindungsgemäßen Verbindungen zur Behandlung von HCMV Infektionen kann im folgenden Tiermodell untersucht werden:

**Tabelle 2: HCMV-Stämme mit Mutationen in UL86 und ihre Empfindlichkeit gegenüber diversen HCMV-UL86-Inhibitoren im Vergleich zum Wildtyp HCMV-AD169**

| IC 50 [µM] | HCMV AD169-Klon mit Mutation in UL86 | | | | | | | | | | | | |
|---|---|---|---|---|---|---|---|---|---|---|---|---|---|
| Substanz | Wildtyp | R435C | D441N | D563N | P586T | V601M | R682H | A689T | P1189T | Q1223R | A1226T | E1320Q | K1338N |
| Ganciclovir | 1 | 1 | 1 | 1 | 1 | 1 | 1 | 1 | 1 | 1 | 1 | 1 | 1 |
| 1 | 1 | 1,5 | 20 | >125 | 4 | 20 | 20 | 6 | >125 | 20 | >125 | >125 | >126 |
| 2 | 0,4 | 0,5 | 1,5 | 6 | 2 | 3 | 3 | 1 | >125 | 6 | >125 | 13 | >126 |
| 3 | 0,5 | >20 | >20 | >20 | >20 | >20 | >20 | >20 | >20 | >20 | >20 | >20 | >20 |
| 4 | 0,08 | >60 | 0.5 | 1 | 0,5 | 0,5 | 0,5 | 0,5 | 2 | 1 | 0,8 | 2 | >60 |
| 5 | 0.14 | 6 | n.d. | 1,4 | 0,8 | 1,5 | n.d. | 0,4 | n.d. | 1,4 | n.d. | 9 | >9 |
| 7 | 1.5 | 3 | n.d. | 16 | 7,6 | 12 | n.d. | 4,5 | n.d. | 12 | n.d. | 24 | 15 |
| 8 | 0,6 | 1.3 | n.d. | >21 | >21 | >21 | n.d. | >21 | n.d. | >21 | n.d. | >21 | >21 |

### HCMV Xenograft-Gelfoam^{®}-Modell

### Tiere:

3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson, USA) bezogen. Die Tiere werden unter sterilen Bedingungen (einschließlich Streu und Futter) in Isolatoren gehalten.

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10 % FKS aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10 % FKS auf einen feuchten Schwamm getropft. 12-13 Stunden später werden die infizierten Schwämme mit 25 µl PBS / 0,1 % BSA / 1 mM DTT mit 5 ng/µl basic Fibroblast Growth Factor (bFGF) inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber verschlossen. 6 Stunden nach der Transplantation werden die Mäuse zum ersten Mal behandelt (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (14 Uhr) peroral mit Substanz behandelt. Die Tagesdosis beträgt 3 oder 10 oder 30 oder 60 oder 100 mg/kg Körpergewicht, das Applikationsvolumen 10 ml/kg Körpergewicht. Die Formulierung der Substanzen erfolgt in Form einer 0,5 %igen Tylosesuspension mit 2 % DMSO oder einer 0,5 %igen Tylosesuspension. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/ 1,5 ml) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wird die Anzahl infektiöser Viruspartikel nach Substanzbehandlung im Vergleich zur placebobehandelten Kontrollgruppe. Für die erfindungsgemäßen Substanzen ergaben sich die in Tabelle 3 aufgeführten ungefähren Ergebnisse:

**Tabelle 3: Antivirale Wirksamkeit in vivo**

| **Beispiel** | **ED50 [mg/kg/Tag]** |
|---|---|
| **1** | 50 |
| **2** | 45 |
| **4** | 70 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

## Patentansprüche

1. Verfahren zum Auswählen von Verbindungen mit anti-Herpesvirus-Aktivität, **gekennzeichnet dadurch, dass**
i) Herpesviren mit Testverbindungen in Kontakt gebracht werden,
ii) resistente Herpesviren ausgelesen werden,
iii) das für das Major Capsid Protein kodierende Gen dieser resistenten Herpesviren sequenziert and die resultierende Proteinsequenz des Major-Capsid-Proteins abgeleitet wird, und
iv) solche Verbindungen ausgewählt werden, bei denen resistente Herpesviren mit einer oder mehreren Aminosäuresubstitutionen im Major-Capsid-Protein auftreten.

2. Verfahren nach Anspruch 1, wobei das Herpesvirus ein Humanes Cytomegalievirus (HCMV) ist.

## Claims

1. A method for selecting compounds comprising anti-herpes virus activity, **characterized in that**
i) herpes viruses are brought into contact with test compounds,
ii) resistant herpes viruses are selected,
iii) the gene encoding the major capsid protein of these resistant herpes viruses is sequenced, and the resulting protein sequence of the major capsid protein is derived,
iv) such compounds are selected with which resistant herpes viruses comprising one or more amino acid substitutions in the major capsid protein occur.

2. The method according to claim 1, whereby the herpes virus is a human cytomegalovirus (HCMV).

## Revendications

1. Procédé de sélection de composés doués d'activité contre les virus herpétiques, **caractérisé en ce que**
i) des herpèsvirus sont mis en contact avec des composés à éprouver,
ii) les herpèsvirus résistants sont séparés par triage,
iii) le gène codant la major-capside-protéine de ces herpèsvirus résistants est séquencé et la séquence protéinique résultante de la major-capside-protéine est déduite, et
iv) des composés pour lesquels apparaissent des herpèsvirus avec une ou plusieurs substitutions d'acides aminés dans la major-capside-protéine sont sélectionnés.

2. Procédé suivant la revendication 1, dans lequel l'herpèsvirus est un cytomégalovirus humain (HCMV).


### SEQUENCE LISTING

<110> Bayer AG, BHC
<120> Methode zur Inhibition der Replikation von Herpesviren
<130> Le A 36 269
<160> 1
<170> Patent In version 3.1
<210> 1
   <211> 1370
   <212> PRT
   <213> Human cytomegalovirus
<400> 1
   Met Glu Asn Trp Ser Ala Leu Glu Leu Leu Pro Lys Val Gly Ile Pro
   1 5 10 15
   Thr Asp Phe Leu Thr His Val Lys Thr Ser Ala Gly Glu Glu Met Phe
   20 25 30
   Glu Ala Leu Arg Ile Tyr Tyr Gly Asp Asp Pro Glu Arg Tyr Asn Ile
   35 40 45
   His Phe Glu Ala Ile Phe Gly Thr Phe Cys Asn Arg Leu Glu Trp Val
   50 55 60
   Tyr Phe Leu Thr Ser Gly Leu Ala Ala Ala Ala His Ala Ile Lys Phe
   65 70 75 80
   His Asp Leu Asn Lys Leu Thr Thr Gly Lys Met Leu Phe His Val Gln
   85 90 95
   Val Pro Arg Val Ala Ser Gly Ala Gly Leu Pro Thr Ser Arg Gln Thr
   100 105 110
   Thr Ile Met Val Thr Lys Tyr Ser Glu Lys Ser Pro Ile Thr Ile Pro
   115 120 125
   Phe Glu Leu Ser Ala Ala Cys Leu Thr Tyr Leu Arg Glu Thr Phe Glu
   130 135 140
   Gly Thr Ile Leu Asp Lys Ile Leu Asn Val Glu Ala Met His Thr Val
   145 150 155 160
   Leu Arg Ala Leu Lys Asn Thr Ala Asp Ala Met Glu Arg Gly Leu Ile
   165 170 175
   His Ser Phe Leu Gln Thr Leu Leu Arg Lys Ala Pro Pro Tyr Phe Val
   180 185 190
   Val Gln Thr Leu Val Glu Asn Ala Thr Leu Ala Arg Gln Ala Leu Asn
   195 200 205
   Arg Ile Gln Arg Ser Asn Ile Leu Gln Ser Phe Lys Ala Lys Met Leu
   210 215 220
   Ala Thr Leu Phe Leu Leu Asn Arg Thr Arg Asp Arg Asp Tyr Val Leu
   225 230 235 240
   Lys Phe Leu Thr Arg Leu Ala Glu Ala Ala Thr Asp Ser Ile Leu Asp
   245 250 255
   Asn Pro Thr Thr Tyr Thr Thr Ser Ser Gly Ala Lys Ile Ser Gly Val
   260 265 270
   Met Val Ser Thr Ala Asn Val Met Gln Ile Ile Met Ser Leu Leu Ser
   275 280 285
   Ser His Ile Thr Lys Glu Thr Val Ser Ala Pro Ala Thr Tyr Gly Asn
   290 295 300
   Phe Val Leu Ser Pro Glu Asn Ala Val Thr Ala Ile Ser Tyr His Ser
   305 310 315 320
   Ile Leu Ala Asp Phe Asn Ser Tyr Lys Ala His Leu Thr Ser Gly Gln
   325 330 335
   Pro His Leu Pro Asn Asp Ser Leu Ser Gln Ala Gly Ala His Ser Leu
   340 345 350
   Thr Pro Leu Ser Met Asp Val Ile Arg Leu Gly Glu Lys Thr Val Ile
   355 360 365
   Met Glu Asn Leu Arg Arg Val Tyr Lys Asn Thr Asp Thr Lys Asp Pro
   370 375 380
   Leu Glu Arg Asn Val Asp Leu Thr Phe Phe Phe Pro Val Gly Leu Tyr
   385 390 395 400
   Leu Pro Glu Asp Arg Gly Tyr Thr Thr Val Glu Ser Lys Val Lys Leu
   405 410 415
   Asn Asp Thr Val Arg Asn Ala Leu Pro Thr Thr Ala Tyr Leu Leu Asn
   420 425 430
   Arg Asp Arg Ala Val Gln Lys Ile Asp Phe Val Asp Ala Leu Lys Thr
   435 440 445
   Leu Cys His Pro Val Leu His Glu Pro Ala Pro Cys Leu Gln Thr Phe
   450 455 460
   Thr Glu Arg Gly Pro Pro Ser Glu Pro Ala Met Gln Arg Leu Leu Glu
   465 470 475 480
   Cys Arg Phe Gln Gln Glu Pro Met Gly Gly Ala Ala Arg Arg Ile Pro
   485 490 495
   His Phe Tyr Arg Val Arg Arg Glu Val Pro Arg Thr Val Asn Glu Met
   500 505 510
   Lys Gln Asp Phe Val Val Thr Asp Phe Tyr Lys Val Gly Asn Ile Thr
   515 520 525
   Leu Tyr Thr Glu Leu His Pro Phe Phe Asp Phe Thr His Cys Gln Glu
   530 535 540
   Asn Ser Glu Thr Val Ala Leu Cys Thr Pro Arg Ile Val Ile Gly Asn
   545 550 555 560
   Leu Pro Asp Gly Leu Ala Pro Gly Pro Phe His Glu Leu Arg Thr Trp
   565 570 575
   Glu Ile Met Glu His Met Arg Leu Arg Pro Pro Pro Asp Tyr Glu Glu
   580 585 590
   Thr Leu Arg Leu Phe Lys Thr Thr Val Thr Ser Pro Asn Tyr Pro Glu
   595 600 605
   Leu Cys Tyr Leu Val Asp Val Leu Val His Gly Asn Val Asp Ala Phe
   610 615 620
   Leu Leu Ile Arg Thr Phe Val Ala Arg Cys Ile Val Asn Met Phe His
   625 630 635 640
   Thr Arg Gln Leu Leu Val Phe Ala His Ser Tyr Ala Leu Val Thr Leu
   645 650 655
   Ile Ala Glu His Leu Ala Asp Gly Ala Leu Pro Pro Gln Leu Leu Phe
   660 665 670
   His Tyr Arg Asn Leu Val Ala Val Leu Arg Leu Val Thr Arg Ile Ser
   675 680 685
   Ala Leu Pro Gly Leu Asn Asn Gly Gln Leu Ala Glu Glu Pro Leu Ser
   690 695 700
   Ala Tyr Val Asn Ala Leu His Asp His Arg Leu Trp Pro Pro Phe Val
   705 710 715 720
   Thr His Leu Pro Arg Asn Met Glu Gly Val Gln Val Val Ala Asp Arg
   725 730 735
   Gln Pro Leu Asn Pro Ala Asn Ile Glu Ala Arg His His Gly Val Ser
   740 745 750
   Asp Val Pro Arg Leu Gly Ala Met Asp Ala Asp Glu Pro Leu Phe Val
   755 760 765
   Asp Asp Tyr Arg Ala Thr Asp Asp Glu Trp Thr Leu Gln Lys Val Phe
   770 775 780
   Tyr Leu Cys Leu Met Pro Ala Met Thr Asn Asn Arg Ala Cys Gly Leu
   785 790 795 800
   Gly Leu Asn Leu Lys Thr Leu Leu Val Asp Leu Phe Tyr Arg Pro Ala
   805 810 815
   Phe Leu Leu Met Pro Ala Ala Thr Ala Val Ser Thr Ser Gly Thr Thr
   820 825 830
   Ser Lys Glu Ser Thr Ser Gly Val Thr Pro Glu Asp Ser Ile Ala Ala
   835 840 845
   Gln Arg Gln Ala Val Gly Glu Met Leu Thr Glu Leu Val Glu Asp Val
   850 855 860
   Ala Thr Asp Ala His Thr Pro Leu Leu Gln Ala Cys Arg Glu Leu Phe
   865 870 875 880
   Leu Ala Val Gln Phe Val Gly Glu His Val Lys Val Leu Glu Val Arg
   885 890 895
   Ala Pro Leu Asp His Ala Gln Arg Gln Gly Leu Pro Asp Phe Ile Ser
   900 905 910
   Arg Gln His Val Leu Tyr Asn Gly Cys Cys Val Val Thr Ala Pro Lys
   915 920 925
   Thr Leu Ile Glu Tyr Ser Leu Pro Val Pro Phe His Arg Phe Tyr Ser
   930 935 940
   Asn Pro Thr Ile Cys Ala Ala Leu Ser Asp Asp Ile Lys Arg Tyr Val
   945 950 955 960
   Thr Glu Phe Pro His Tyr His Arg His Asp Gly Gly Phe Pro Leu Pro
   965 970 975
   Thr Ala Phe Ala His Glu Tyr His Asn Trp Leu Arg Ser Pro Phe Ser
   980 985 990
   Arg Tyr Ser Ala Thr Cys Pro Asn Val Leu His Ser Val Met Thr Leu
   995 1000 1005
   Ala Ala Met Leu Tyr Lys Ile Ser Pro Val Ser Leu Val Leu Gln
   1010 1015 1020
   Thr Lys Ala His Ile His Pro Gly Phe Ala Leu Thr Ala Val Arg
   1025 1030 1035
   Thr Asp Thr Phe Glu Val Asp Met Leu Leu Tyr Ser Gly Lys Ser
   1040 1045 1050
   Cys Thr Ser Val Ile Ile Asn Asn Pro Ile Val Thr Lys Glu Glu
   1055 1060 1065
   Arg Asp Ile Ser Thr Thr Tyr His Val Thr Gln Asn Ile Asn Thr
   1070 1075 1080
   Val Asp Met Gly Leu Gly Tyr Thr Ser Asn Thr Cys Val Ala Tyr
   1085 1090 1095
   Val Asn Arg Val Arg Thr Asp Met Gly Val Arg Val Gln Asp Leu
   1100 1105 1110
   Phe Arg Val Phe Pro Met Asn Val Tyr Arg His Asp Glu Val Asp
   1115 1120 1125
   Arg Trp Ile Arg His Ala Ala Gly Val Glu Arg Pro Gln Leu Leu
   1130 1135 1140
   Asp Thr Glu Thr Ile Ser Met Leu Thr Phe Gly Ser Met Ser Glu
   1145 1150 1155
   Arg Asn Ala Ala Ala Thr Val His Gly Gln Lys Ala Ala Cys Glu
   1160 1165 1170
   Leu Ile Leu Thr Pro Val Thr Met Asp Val Asn Tyr Phe Lys Ile
   1175 1180 1185
   Pro Asn Asn Pro Arg Gly Arg Ala Ser Cys Met Leu Ala Val Asp
   1190 1195 1200
   Pro Tyr Asp Thr Glu Ala Ala Thr Lys Ala Ile Tyr Asp His Arg
   1205 1210 1215
   Glu Ala Asp Ala Gln Thr Phe Ala Ala Thr His Asn Pro Trp Ala
   1220 1225 1230
   Ser Gln Ala Gly Cys Leu Ser Asp Val Leu Tyr Asn Thr Arg His
   1235 1240 1245
   Arg Glu Arg Leu Gly Tyr Asn Ser Lys Phe Tyr Ser Pro Cys Ala
   1250 1255 1260
   Gln Tyr Phe Asn Thr Glu Glu Ile Ile Ala Ala Asn Lys Thr Leu
   1265 1270 1275
   Phe Lys Thr Ile Asp Glu Tyr Leu Leu Arg Ala Lys Asp Cys Ile
   1280 1285 1290
   Arg Gly Asp Thr Asp Thr Gln Tyr Val Cys Val Glu Gly Thr Glu
   1295 1300 1305
   Gln Leu Ile Glu Asn Pro Cys Arg Leu Thr Gln Glu Ala Leu Pro
       1310                1315                1320
   Ile Leu Ser Thr Thr Thr Leu Ala Leu Met Glu Thr Lys Leu Lys
       1325                1330                1335
   Gly Gly Ala Gly Ala Phe Ala Thr Ser Glu Thr His Phe Gly Asn
       1340                1345                1350
   Tyr Val Val Gly Glu Ile Ile Pro Leu Gln Gln Ser Met Leu Phe
       1355                1360                1365
   Asn Ser
       1370